# EUROPEAN PATENT SPECIFICATION

(11) **EP 3 185 590 B1**
(45) Date of publication and mention of the grant of the patent: **19.08.2020**
(21) Application number: 16205776.4
(22) Date of filing: 21.12.2016
(51) Int. Cl.: H04R 25/00, A61B 5/0478, A61B 5/00, G06F 3/01

(54) **A HEARING DEVICE COMPRISING A SENSOR FOR PICKING UP ELECTROMAGNETIC SIGNALS FROM THE BODY**
HÖRGERÄT MIT EINEM SENSOR ZUM AUFNEHMEN ELEKTROMAGNETISCHER SIGNALE AUS DEM KÖRPER
DISPOSITIF AUDITIF COMPRENANT UN CAPTEUR SERVANT À CAPTER DES SIGNAUX ÉLECTROMAGNÉTIQUES PROVENANT DU CORPS

(30) Priority: 22.12.2015 EP 15201827
(43) Date of publication of application: 28.06.2017
(73) Proprietor: Oticon A/S, 2765 Smørum (DK)
(72) Inventor: LUNNER, Thomas, 2765 Smørum (DK); GUSTAFSSON, Fredrik, 581 83 Linköping (SE)
(74) Representative: Demant

(56) References cited:
- WO-A1-2009/053184
- US-A- 5 726 916
- US-A1- 2014 198 936
- US-A1- 2014 369 537
- HARLAND C J ET AL: "Electric potential probes - new directions in the remote sensing of the human body; Electric potential probes", MEASUREMENT SCIENCE AND TECHNOLOGY, IOP, BRISTOL, GB, vol. 13, no. 2, 1 February 2002 (2002-02-01), pages 163-169, XP020063427, ISSN: 0957-0233, DOI: 10.1088/0957-0233/13/2/304
- MILTON SEVERO ET AL: "Change Detection with Kalman Filter and CUSUM", 1 June 2010 (2010-06-01), UBIQUITOUS KNOWLEDGE DISCOVERY, SPRINGER BERLIN HEIDELBERG, BERLIN, HEIDELBERG, PAGE(S) 148 - 162, XP019154537, ISBN: 978-3-642-16391-3 * the whole document *
- KALMAN R E: "A NEW APPROACH TO LINEAR FILTERING AND PREDICTION PROBLEMS", TRANSACTIONS OF THE AMERICAN SOCIETY OF MECHANICAL ENGINEERS,SERIES D: JOURNAL OF BASIC ENGINEERING, AMERICAN SOCIETY OF MECHANICAL ENGINEERS, NEW YORK, NY, US, vol. 82, 1 March 1960 (1960-03-01), pages 35-45, XP008039411, ISSN: 0021-9223

## Description

### SUMMARY

The present application relates to a hearing system as defined by claim 1. Additional embodiments are recited in the dependent claims. The disclosure deals with the use of electric potential sensors (EPS), in a hearing device to pick up signals from a user's body, e.g. Electroencephalography (EEG) signals and/or Electroocculography (EOG) signals from the ears or ear canal(s) of a user. In an embodiment, such so-called EarEEG or EarEOG signals are used to control a hearing aid and/or other devices, e.g. accessory devices in communication with the hearing aid. A hearing device which uses electric potential sensors to monitor eye gaze direction is disclosed in US 2014/198936.

In the course of the description, all further occurrences of the words "embodiment(s)" or "aspect(s)", except the ones related to the claims, refer to examples useful for understanding the invention which were originally filed but which do not represent embodiments of the presently claimed invention. These examples are shown for illustrative purposes only.

### A hearing device comprising a sensor part:

In an aspect of the present application, a hearing device, e.g. a hearing aid, comprising a sensor part adapted for being located at or in an ear, or for fully or partially for being implanted in the head, of a user is provided.

In an embodiment, the sensor is configured to sense bioelectric signals due to eye movements, e.g. muscular contraction or changes of the electric eye-field potentials due to eye-bulb rotations, or eye gaze, or due to brain activity, e.g. evoked potentials due to nerve excitation or brainwave signals.

In an embodiment, the sensor part comprises an electrical potential sensor for sensing an electrical potential, and the hearing device further comprises electronic circuitry coupled to the electrical potential sensor to provide an amplified output.

In an embodiment, the electrical potential sensor is configured to sense brain wave signals. Thereby an improved hearing device may be provided.

In an embodiment, the electromagnetic sensor sensor comprises a sensing device configured to be capacitively or inductively coupled to the surface of the user's head, when the hearing device is operatively mounted on the user.

### A hearing device comprising a sensor, e.g. an electric potential sensor:

In an aspect of the present application, a hearing device, e.g. a hearing aid, having a sensor part adapted for being located at or in an ear or for fully or partially for being implanted in the head of a user is provided. The hearing device comprises e.g. an electrical potential sensor for sensing an electrical potential, and electronic circuitry coupled to the electrical potential sensor to provide an amplified output. In an embodiment, the electrical potential sensor is configured to sense brain wave signals originating from neural activity in the user's brain.

In an embodiment, the electrical potential sensor comprises a sensing electrode configured to be coupled to the surface of the user's head (e.g. at or around an ear or in an ear canal), when the hearing device is operatively mounted on the user. In an embodiment, the electrical potential sensor comprises a sensing electrode configured to be capacitively coupled to the surface of the user's head, when the hearing device is operatively mounted on the user. In an embodiment, the electrical potential sensor comprises a sensing electrode configured to be directly (e.g. electrically (galvanically)) coupled to the surface of the user's head (e.g. via a 'dry' or 'wet' contact area between the skin of the user and the (electrically conducting) sensing electrode), when the hearing device is operatively mounted on the user.

In an embodiment, the sensing electrode comprises an electrical conductor and a dielectric material configured to provide said capacitive coupling to the user's head, when the hearing device is operatively mounted on the user.

In an embodiment, the electronic circuitry comprises a bias current path connected to a reference voltage of the sensor part and to the sensing electrode.

In an embodiment, the electronic circuitry comprises a voltage follower circuit coupled to the sensing electrode and to the bias current path, the voltage follower circuit comprising first and second inputs and an output.

In an embodiment, the electrical potential sensor comprises a guard conductor for shielding the sensing electrode. In an embodiment, the guard conductor is coupled to the output of the voltage follower circuit.

In an embodiment, the bias current path and said sensing electrode are coupled to said first input, and wherein said output is coupled to said second input of said voltage follower circuit.

In an embodiment, the hearing device, e.g. the electronic circuitry, is configured to provide a reference potential. In an embodiment, the electronic circuitry comprises a low noise amplifier arranged to amplify the electrical potential relative to the reference potential to provide the amplified output in the form of an amplified voltage (e.g. a digitized voltage). In an embodiment, the hearing device comprises a reference electrode for providing a reference potential.

In an embodiment, the electric circuitry comprises a feedback impedance coupled between said output and said first input of said voltage follower circuit.

In an embodiment, the electric circuitry comprises an amplifier arranged to amplify said output of said voltage follower circuit to provide said amplified output. In an embodiment, the electric circuitry (e.g. the amplifier) comprises an analog to digital converter to provide a digital output from the electric circuitry.

In an embodiment, the hearing device comprises a hearing aid, a headset, an earphone, an ear protection device or a combination thereof.

In an embodiment, the hearing device is adapted to provide a frequency dependent gain and/or a level dependent compression and/or a transposition (with or without frequency compression) of one or frequency ranges to one or more other frequency ranges, e.g. to compensate for a hearing impairment of a user. In an embodiment, the hearing device comprises a signal processing unit for enhancing the input signals and providing a processed output signal. Various aspects of digital hearing aids are described in [Schaub; 2008].

In an embodiment, the hearing device comprises an output unit for providing a stimulus perceived by the user as an acoustic signal based on a processed electric signal. In an embodiment, the output unit comprises a number of electrodes of a cochlear implant or a vibrator of a bone conducting hearing device. In an embodiment, the output unit comprises an output transducer. In an embodiment, the output transducer comprises a receiver (loudspeaker) for providing the stimulus as an acoustic signal to the user. In an embodiment, the output transducer comprises a vibrator for providing the stimulus as mechanical vibration of a skull bone to the user (e.g. in a bone-attached or bone-anchored hearing device).

In an embodiment, the hearing device comprises an input unit for providing an electric input signal representing sound. In an embodiment, the input unit comprises an input transducer for converting an input sound to an electric input signal. In an embodiment, the input unit comprises a wireless receiver for receiving a wireless signal comprising sound and for providing an electric input signal representing said sound. In an embodiment, the hearing device comprises a directional microphone system adapted to enhance a target acoustic source among a multitude of acoustic sources in the local environment of the user wearing the hearing device. In an embodiment, the directional system is adapted to detect (such as adaptively detect) from which direction a particular part of the microphone signal originates. This can be achieved in various different ways as e.g. described in the prior art.

In an embodiment, the hearing device comprises an antenna and transceiver circuitry for wirelessly receiving a direct electric input signal from another device, e.g. a communication device or another hearing device. In an embodiment, the hearing device comprises a (possibly standardized) electric interface (e.g. in the form of a connector) for receiving a wired direct electric input signal from another device, e.g. a communication device or another hearing device. In an embodiment, the direct electric input signal represents or comprises an audio signal and/or a control signal and/or an information signal. In an embodiment, the hearing device comprises demodulation circuitry for demodulating the received direct electric input to provide the direct electric input signal representing an audio signal and/or a control signal e.g. for setting an operational parameter (e.g. volume) and/or a processing parameter of the hearing device. In general, the wireless link established by a transmitter and antenna and transceiver circuitry of the hearing device can be of any type. In an embodiment, the wireless link is used under power constraints, e.g. in that the hearing device comprises a portable (typically battery driven) device. In an embodiment, the wireless link is a link based on near-field communication, e.g. an inductive link based on an inductive coupling between antenna coils of transmitter and receiver parts. In another embodiment, the wireless link is based on farfield, electromagnetic radiation. In an embodiment, the communication via the wireless link is arranged according to a specific modulation scheme, e.g. an analogue modulation scheme, such as FM (frequency modulation) or AM (amplitude modulation) or PM (phase modulation), or a digital modulation scheme, such as ASK (amplitude shift keying), e.g. On-Off keying, FSK (frequency shift keying), PSK (phase shift keying) or QAM (quadrature amplitude modulation).

In an embodiment, the communication between the hearing device and the other device is in the base band (audio frequency range, e.g. between 0 and 20 kHz). Preferably, communication between the hearing device and the other device is based on some sort of modulation at frequencies above 100 kHz. Preferably, frequencies used to establish a communication link between the hearing device and the other device is below 50 GHz, e.g. located in a range from 50 MHz to 50 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). In an embodiment, the wireless link is based on a standardized or proprietary technology. In an embodiment, the wireless link is based on Bluetooth technology (e.g. Bluetooth Low-Energy technology).
In an embodiment, the hearing device is portable device, e.g. a device comprising a local energy source, e.g. a battery, e.g. a rechargeable battery.

In an embodiment, the hearing device comprises a forward or signal path between an input transducer (microphone system and/or direct electric input (e.g. a wireless receiver)) and an output transducer. In an embodiment, the signal processing unit is located in the forward path. In an embodiment, the signal processing unit is adapted to provide a frequency dependent gain according to a user's particular needs. In an embodiment, the hearing device comprises an analysis path comprising functional components for analyzing the input signal (e.g. determining a level, a modulation, a type of signal, an acoustic feedback estimate, etc.). In an embodiment, some or all signal processing of the analysis path and/or the signal path is conducted in the frequency domain. In an embodiment, some or all signal processing of the analysis path and/or the signal path is conducted in the time domain.

In an embodiment, an analogue electric signal representing an acoustic signal is converted to a digital audio signal in an analogue-to-digital (AD) conversion process, where the analogue signal is sampled with a predefined sampling frequency or rate fₛ, fₛ being e.g. in the range from 8 kHz to 40 kHz (adapted to the particular needs of the application) to provide digital samples Xₙ (or x[n]) at discrete points in time tₙ (or n), each audio sample representing the value of the acoustic signal at tₙ by a predefined number Nₛ of bits, Nₛ being e.g. in the range from 1 to 16 bits. A digital sample x has a length in time of 1/fₛ, e.g. 50 µs, for *f*s = 20 kHz. In an embodiment, a number of audio samples are arranged in a time frame. In an embodiment, a time frame comprises 64 audio data samples. Other frame lengths may be used depending on the practical application.

In an embodiment, the hearing devices comprise an analogue-to-digital (AD) converter to digitize an analogue input with a predefined sampling rate, e.g. 20 kHz. In an embodiment, the hearing devices comprise a digital-to-analogue (DA) converter to convert a digital signal to an analogue output signal, e.g. for being presented to a user via an output transducer.

In an embodiment, the hearing device, e.g. the microphone unit, and or the transceiver unit comprise(s) a TF-conversion unit for providing a time-frequency representation of an input signal. In an embodiment, the time-frequency representation comprises an array or map of corresponding complex or real values of the signal in question in a particular time and frequency range. In an embodiment, the TF conversion unit comprises a filter bank for filtering a (time varying) input signal and providing a number of (time varying) output signals each comprising a distinct frequency range of the input signal. In an embodiment, the TF conversion unit comprises a Fourier transformation unit for converting a time variant input signal to a (time variant) signal in the frequency domain. In an embodiment, the frequency range considered by the hearing device from a minimum frequency fₘᵢₙ to a maximum frequency fₘₐₓ comprises a part of the typical human audible frequency range from 20 Hz to 20 kHz, e.g. a part of the range from 20 Hz to 12 kHz. In an embodiment, a signal of the forward and/or analysis path of the hearing device is split into a number *NI* of frequency bands, where NI is e.g. larger than 5, such as larger than 10, such as larger than 50, such as larger than 100, such as larger than 500, at least some of which are processed individually. In an embodiment, the hearing device is/are adapted to process a signal of the forward and/or analysis path in a number *NP* of different frequency channels (*NP* ≤ *NI*)*.* The frequency channels may be uniform or non-uniform in width (e.g. increasing in width with frequency), overlapping or non-overlapping.

In an embodiment, the hearing device comprises a level detector (LD) for determining the level of an input signal (e.g. on a band level and/or of the full (wide band) signal). The input level of the electric microphone signal picked up from the user's acoustic environment is e.g. a classifier of the environment. In an embodiment, the level detector is adapted to classify a current acoustic environment of the user according to a number of different (e.g. average) signal levels, e.g. as a HIGH-LEVEL or LOW-LEVEL environment.

In a particular embodiment, the hearing device comprises a voice detector (VD) for determining whether or not an input signal comprises a voice signal (at a given point in time). A voice signal is in the present context taken to include a speech signal from a human being. It may also include other forms of utterances generated by the human speech system (e.g. singing). In an embodiment, the voice detector unit is adapted to classify a current acoustic environment of the user as a VOICE or NO-VOICE environment. This has the advantage that time segments of the electric microphone signal comprising human utterances (e.g. speech) in the user's environment can be identified, and thus separated from time segments only comprising other sound sources (e.g. artificially generated noise). In an embodiment, the voice detector is adapted to detect as a VOICE also the user's own voice. Alternatively, the voice detector is adapted to exclude a user's own voice from the detection of a VOICE.

In an embodiment, the hearing device comprises an own voice detector for detecting whether a given input sound (e.g. a voice) originates from the voice of the user of the system. In an embodiment, the microphone system of the hearing device is adapted to be able to differentiate between a user's own voice and another person's voice and possibly from NON-voice sounds.

In an embodiment, the hearing device further comprises other relevant functionality for the application in question, e.g. feedback suppression, compression, noise reduction, etc.

### Use:

In an aspect, use of a hearing device as described above, in the 'detailed description of embodiments' and in the claims, is moreover provided.

### A hearing system:

In a further aspect, a hearing system comprising a hearing device as described above, in the 'detailed description of embodiments', and in the claims, AND an auxiliary device is moreover provided.

In an embodiment, the system is adapted to establish a communication link between the hearing device and the auxiliary device to provide that information (e.g. control and status signals, possibly audio signals) can be exchanged or forwarded from one to the other.

In an embodiment, the auxiliary device is or comprises an audio gateway device adapted for receiving a multitude of audio signals (e.g. from an entertainment device, e.g. a TV or a music player, a telephone apparatus, e.g. a mobile telephone or a computer, e.g. a PC) and adapted for selecting and/or combining an appropriate one of the received audio signals (or combination of signals) for transmission to the hearing device. In an embodiment, the auxiliary device is or comprises a remote control for controlling functionality and operation of the hearing device(s). In an embodiment, the auxiliary device is or comprises a smartphone or similar device, e.g. a smartwatch. In an embodiment, the function of a remote control is implemented in a SmartPhone (or smartwatch), the SmartPhone (or smartwatch) possibly running an APP allowing to control the functionality of the audio processing device via the SmartPhone (the hearing device(s) comprising an appropriate wireless interface to the SmartPhone, e.g. based on Bluetooth or some other standardized or proprietary scheme).

In an embodiment, the auxiliary device is or comprises another hearing device. In an embodiment, the hearing system comprises two hearing devices adapted to implement a binaural hearing system, e.g. a binaural hearing aid system.

In an embodiment, the hearing device and the auxiliary device are configured to allow an exchange of data between them, including EarEEG signals.

In an embodiment, the auxiliary device comprises a further hearing device as described above, in the 'detailed description of embodiments', and in the claims. The hearing system thereby comprises left and right hearing devices adapted for being located at or in left and right ears and/or for fully or partially for being implanted in the head at left and right ears of a user. In an embodiment, the hearing system comprises left and right hearing devices as described above, in the 'detailed description of embodiments', and in the claims, and a further auxiliary device, e.g. a remote control device comprising a user interface and optionally a further processing capability. The user interface may e.g. be implemented as an APP, e.g. of a smartphone, tablet computer, a smartwatch or similar device.

In an embodiment, the hearing system is configured to allow at least one of the hearing devices to generate an ear EEG and/or an ear EOG signal. In an embodiment, the hearing system is configured to allow at least one of the hearing devices or a further auxiliary device to generate an ear EEG and/or an ear EOG signal based on ear EEG and/or an ear EOG signal from said left and right hearing devices.

In an embodiment, the hearing device and the auxiliary device (e.g. another hearing device and/or a separate processing or relaying device, e.g. a smartphone or the like) are configured to allow an exchange of data between them, including said amplified output, or signals based thereon, e.g. EarEEG and/or EarEOG signals. In an embodiment, the hearing system is configured to allow of exchange of data between the left and right hearing devices, either directly or via a (further) auxiliary device, e.g. a remote control or a smartphone or the like.

In an embodiment, at least one of the hearing devices of the hearing system is configured to allow the reception of audio signals from a multitude of audio sources, e.g. wireless microphones, and comprises a control unit for selecting one of the audio signals in dependence of an ear EEG and/or an EarEOG control signal.

In an embodiment, at least one of the hearing devices comprises a beamformer unit, and said at least one hearing device is configured to control the beamformer unit, or a binaural beamformer unit (where beamforming is coordinated between the left and right hearing devices), in dependence of an ear EEG and/or an EarEOG control signal.

In an embodiment, the hearing system comprises an interface to a computer or a smartphone wherein at least one of the hearing devices is configured to control the interface, e.g. a mouse function, in dependence of an ear EEG and/or an EarEOG control signal.

In an embodiment, the hearing system, e.g. the at least one hearing device, or a separate unit, comprises a reference electrode for providing a reference potential (P₀).

In an embodiment, the hearing system is configured to sense over time, a) left and right EPS sense potentials P_{left} and P_{right}, respectively, and b) to compare the sensed potentials to a reference potential P₀, and c) to provide respective amplified voltages V_{left} = A(P_{left} - P₀) and V_{right} = A(P_{right} - P₀), where A is an amplification factor.

In an embodiment, the left and right amplified voltages V_{left} and V_{right} are representative of respective left and right EEG signals, termed EarEEG_{left} and EarEEG_{right}, respectively, and wherein - in an eye gaze detection mode - the measured potentials V_{left} and V_{right} are representative of eye movement (e.g. an eye gaze angle or direction), and wherein the hearing system is configured to transmit one of the left and right amplified voltages V_{left} and V_{right} to the respective other hearing device, or to another device, or to exchange said amplified voltages between the left and right hearing devices, or to transmit said amplified voltages to another device.

In an embodiment, an EarEOG signal representative of eye gaze direction is determined based on the left and right amplified voltages V_{left} and V_{right}.

In an embodiment, the EarEOG signal is a function (f) of a difference between the left and right amplified voltages V_{left} and V_{right}, EarEOG=f(V_{left} - V_{right}).

In an embodiment, the hearing system comprises a processing unit configured to provide an EarEOG control signal for controlling a function of said at least one hearing device based on said EarEOG signal(s).

In an embodiment, the hearing system comprises a location sensor unit (e.g. a head tracker, e.g. based on linear acceleration (accelerometer) and/or angular acceleration (gyroscope) data) for providing location data representative of a current location of the user, and a calculation unit configured to combine said location data with said EEG and/or ear EOG signal(s) to provide combined location data.

In an embodiment, the hearing system comprises a linear adaptive filter and a nonlinear change detector configured to be used for filtering of said amplified output, or signals based thereon. In an embodiment, the linear adaptive filter comprises a Kalman filter. In an embodiment, the nonlinear change detector is based on a CUSUM algorithm.

In an embodiment, the hearing system comprises a Kalman filter for filtering said location data, and/or said ear EEG and/or ear EOG signal(s), and/or said combined location data, and providing eye gaze angles or eye gaze directions in a fixed coordinate system. In an embodiment, the hearing system comprises a Kalman filter for filtering said combined location data and providing absolute coordinates of an object, e.g. a sound source, which is of current interest to the user. In an embodiment, the hearing system comprises a Kalman filter and a change detector (e.g. a CUSUM detector) configured to be used for filtering of said amplified output, or signals based thereon. In an embodiment, the calculation unit is configured to determine locations of hotspots representing preferred eye gaze directions of the user based on said combined location data. Thereby a spatial map of currently interesting areas ('hotspots') for the user can be identified. In an embodiment, the hearing system (e.g. the individual hearing devices or other auxiliary devices) is configured to use said spatial map of currently (acoustically) interesting areas to simplify processing, e.g. by calculate fixed beamformers of a beamformer filtering unit for at least some of the identified hotspots (e.g. the most significant ones) to be applied when a given hotspot is estimated to be of the user's current interest (according to the current combined location data, e.g. based on eye gaze).

### An APP:

In a further aspect, a non-transitory storage medium storing a processor-executable program that, when executed by a processor of an auxiliary device, implements a user interface process for a binaural hearing system including left and right hearing devices is provided by the present disclosure. The process comprises:
- exchanging information with the left and right hearing assistance devices;
- providing a graphical interface configured to illustrate one or more current sound sources relative to the user; and
- illustrating at least one of said current sound sources as being selected by the user by eye gaze for being presented to the user at least one of the left and right hearing devices.

In an embodiment, processor-executable program on the non-transitory storage medium is configured to
- executing, based on input from a user via the user interface, at least one of:
   ▪ adding a further one said current sound sources for being presented to the user at least one of the left and right hearing devices; and
   ▪ substituting said current sound source being selected by the user by eye gaze with another one of said current sound sources;
   ▪ adjusting a volume of the selected current sound source(s).

In an embodiment, processor-executable program (here termed an 'APP') that, when executed by a processor of an auxiliary device, implements a user interface process for the binaural hearing system described above in the 'detailed description of embodiments', and in the claims. In an embodiment, the APP is configured to run on cellular phone, e.g. a smartphone, or on another portable device allowing communication with said hearing device or said hearing system (including on the hearing device).

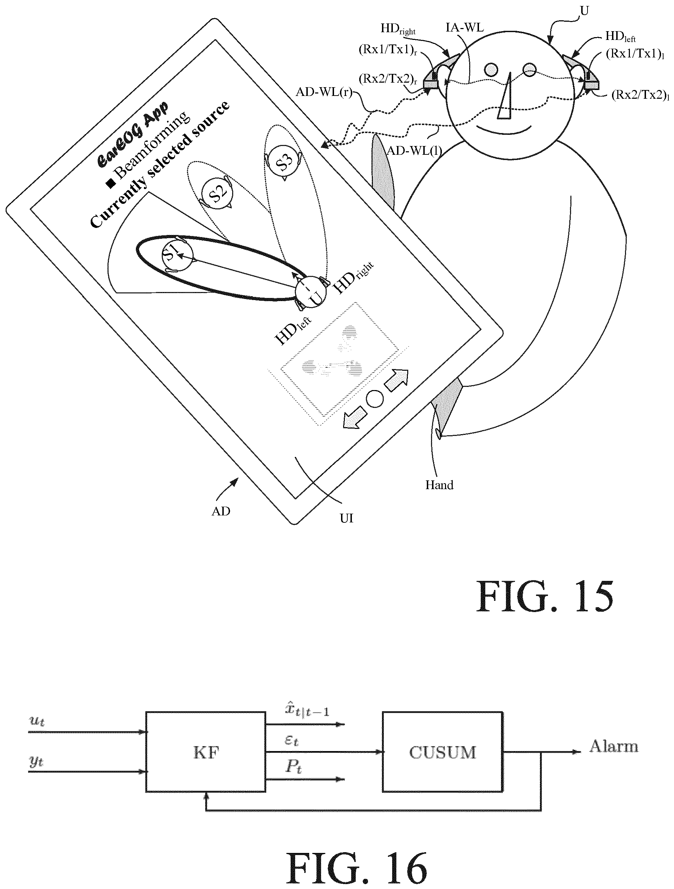

In an embodiment, the APP is configured to display a currently available set of sound sources of interest to a user. In an embodiment, the APP is configured to display a current selection by eye gaze of a sound source of interest among a number of available sound sources (cf. *EarEOG APP* in FIG. 15).

### Definitions:

In the present context, a 'hearing device' refers to a device, such as e.g. a hearing instrument or an active ear-protection device or other audio processing device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. A 'hearing device' further refers to a device such as an earphone or a headset adapted to receive audio signals electronically, possibly modifying the audio signals and providing the possibly modified audio signals as audible signals to at least one of the user's ears. Such audible signals may e.g. be provided in the form of acoustic signals radiated into the user's outer ears, acoustic signals transferred as mechanical vibrations to the user's inner ears through the bone structure of the user's head and/or through parts of the middle ear as well as electric signals transferred directly or indirectly to the cochlear nerve of the user.

The hearing device may be configured to be worn in any known way, e.g. as a unit arranged behind the ear with a tube leading radiated acoustic signals into the ear canal or with a loudspeaker arranged close to or in the ear canal, as a unit entirely or partly arranged in the pinna and/or in the ear canal, as a unit attached to a fixture implanted into the skull bone, as an entirely or partly implanted unit, etc. The hearing device may comprise a single unit or several units communicating electronically with each other.

More generally, a hearing device comprises an input transducer for receiving an acoustic signal from a user's surroundings and providing a corresponding input audio signal and/or a receiver for electronically (i.e. wired or wirelessly) receiving an input audio signal, a (typically configurable) signal processing circuit for processing the input audio signal and an output unit for providing an audible signal to the user in dependence on the processed audio signal. The signal processing unit may be adapted to process the input signal in the time domain or in a number of frequency bands. In some hearing devices, an amplifier and/or compressor may constitute the signal processing circuit. The signal processing circuit typically comprises one or more (integrated or separate) memory elements for executing programs and/or for storing parameters used (or potentially used) in the processing and/or for storing information relevant for the function of the hearing device and/or for storing information (e.g. processed information, e.g. provided by the signal processing circuit), e.g. for use in connection with an interface to a user and/or an interface to a programming device. In some hearing devices, the output unit may comprise an output transducer, such as e.g. a loudspeaker for providing an airborne acoustic signal or a vibrator for providing a structure-borne or liquid-borne acoustic signal. In some hearing devices, the output unit may comprise one or more output electrodes for providing electric signals (e.g. a multi-electrode array for electrically stimulating the cochlear nerve).

In some hearing devices, the vibrator may be adapted to provide a structure-borne acoustic signal transcutaneously or percutaneously to the skull bone. In some hearing devices, the vibrator may be implanted in the middle ear and/or in the inner ear. In some hearing devices, the vibrator may be adapted to provide a structure-borne acoustic signal to a middle-ear bone and/or to the cochlea. In some hearing devices, the vibrator may be adapted to provide a liquid-borne acoustic signal to the cochlear liquid, e.g. through the oval window. In some hearing devices, the output electrodes may be implanted in the cochlea or on the inside of the skull bone and may be adapted to provide the electric signals to the hair cells of the cochlea, to one or more hearing nerves, to the auditory cortex and/or to other parts of the cerebral cortex.

A hearing device, e.g. a hearing aid, may be adapted to a particular user's needs, e.g. a hearing impairment. A configurable signal processing circuit of the hearing device may be adapted to apply a frequency and level dependent compressive amplification of an input signal. A customized frequency and level dependent gain may be determined in a fitting process by a fitting system based on a user's hearing data, e.g. an audiogram, using a fitting rationale. The frequency and level dependent gain may e.g. be embodied in processing parameters, e.g. uploaded to the hearing device via an interface to a programming device (fitting system), and used by a processing algorithm executed by the configurable signal processing circuit of the hearing device.

A 'hearing system' refers to a system comprising one or two hearing devices, and a 'binaural hearing system' refers to a system comprising two hearing devices and being adapted to cooperatively provide audible signals to both of the user's ears. Hearing systems or binaural hearing systems may further comprise one or more 'auxiliary devices', which communicate with the hearing device(s) and affect and/or benefit from the function of the hearing device(s). Auxiliary devices may be e.g. remote controls, audio gateway devices, mobile phones (e.g. SmartPhones), or music players. Hearing devices, hearing systems or binaural hearing systems may e.g. be used for compensating for a hearing-impaired person's loss of hearing capability, augmenting or protecting a normal-hearing person's hearing capability and/or conveying electronic audio signals to a person. Hearing devices or hearing systems may e.g. form part of or interact with public-address systems, active ear protection systems, handsfree telephone systems, car audio systems, entertainment (e.g. karaoke) systems, teleconferencing systems, classroom amplification systems, etc.

Embodiments of the disclosure may e.g. be useful in applications such as hearing aids, headsets, ear phones, active ear protection systems or combinations thereof.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 shows a schematic diagram of an EPS with associated electronic circuitry: An internal current is generated that is modulated by the electrical field,
FIG. 2 shows an embodiment of an electric potential sensor,
FIG. 3A and 3B illustrate the use of electrooculography (EOG) to detect eye movement, FIG. 3A showing an EOG signal detecting an eye-movement of the eyes to the right, FIG. 3B showing an EOG signal detecting an eye-movement to the left,
FIG. 4 shows EarEEG signals picked up in the left and right ears and subtracted from each other and post processed to remove a DC component to create the EarEOG signal (curve, labelled 'EarEEG'), and this signal is compared to an eye-tracker signal based on infrared tracking of the eye gaze (curve, labelled 'EyeTribe'),
FIG. 5 shows a pair of behind the ear (BTE) hearing devices with EEG electrodes on the mould surface,
FIG. 6 schematically illustrates an EPS electrode in left and right ears, respectively, wherein the EarEEG signals picked up at one EPS electrode at one ear is transmitted to the other ear to create a difference signal, whereby EOG in the ear, EarEOG, is provided,
FIG. 7 illustrates an application scenario of a hearing device or a pair of hearing devices according to the present disclosure using EarEOG in a situation with multiple talkers,
FIG. 8 illustrates a situation of use EarEOG control of a beamformer of a hearing device according to the present disclosure,
FIG. 9 shows an example of individually 3D-printed ear mould made in stainless steel,
FIG. 10 shows a use scenario of an embodiment of a hearing system comprising EEG and reference electrodes according to the present disclosure,
FIG. 11 shows an embodiment of a hearing system comprising a hearing device and an auxiliary device in communication with each other, and
FIG. 12 illustrates a method of providing data for controlling functionality in a head worn hearing device, e.g. a hearing aid,
FIG. 13A illustrates a first embodiment of a hearing device according to the present disclosure, and
FIG. 13B illustrates a second embodiment of a hearing device according to the present disclosure,
FIG. 14 shows an embodiment of a binaural hearing system comprising left and right hearing devices and an auxiliary device in communication with each other according to the present disclosure,
FIG. 15 shows use of a binaural hearing system comprising left and right hearing devices and an auxiliary device in communication with each other, the auxiliary device comprising an APP implementing a user interface according to the present disclosure for the hearing system, and
FIG. 16 illustrates the use of a Kalman filter together with a change detector to detect fast changes in gaze angle, and
FIG. 17A shows eye gaze signals, up-down (top), and left-right (bottom), from a camera, as a function of time (samples).
FIG. 17B shows the eye gaze direction as obtained from the data of FIG. 17A in a pan-tilt plane to illustrate the two dimensional points of fixation.

The figures are schematic and simplified for clarity, and they just show details which are essential to the understanding of the disclosure, while other details are left out.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practised without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

### EarEEG electrodes

Conventional Electroencephalography (EEG) requires contact with skin and uses at least two electrodes to create a closed circuit, an active and a reference electrode. Thereby a small but detectable current flows from the brain to the pickup device (e.g. an operational amplifier) is created and a voltage difference can be read. By taking the difference between the potential of the two electrodes the EEG signal can be detected.

This is also the case when EEG is read from the ear canal in the form of EarEEG, where the electrodes are contained in a hearing aid earmould, a dome or at the surface of the hearing aid shell.

The problem for EarEEG is that, with the limited physical distance between the active and reference electrodes, the resulting difference signal is either a) lower than conventional EEG since two electrodes rather close to each other picks up similar electrical activity from the brain, or b) that a communication channel (e.g. a cable) between the two hearing aids is required to create a reference with larger distance from the active electrode.

Another problem for EEG or EarEEG is that they both require a physical contact with the skin for creating the small (but detectable) current. This creates problems requiring wet electrodes with conducting paste contact paste or dry electrodes with high impedance that can induce movement artefacts.

### Electric Potential Sensors

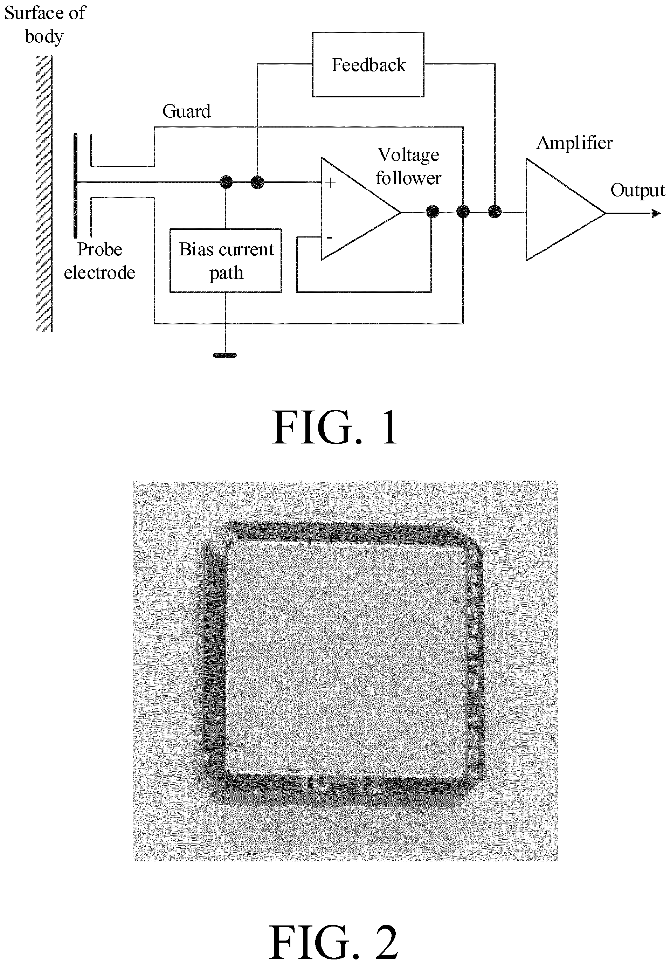

The present disclosure deals (among other things) with the use of electrical potential sensors in hearing devices to overcome the above mentioned problems. An Electric Potential Sensor (EPS) is a device that senses the electric field variations rather than sensing (small) electrical currents. This requires only one active electrode to create a readable voltage. Furthermore, direct skin contact is not necessary to sense the electric field. FIG. 1 shows a schematic diagram of an EPS with associated electronic circuitry, termed an electronic potential integrated circuit (EPIC), to pick up and amplify the electric potential from the brain.

FIG. 1 shows a schematic diagram of an EPS with associated electronic circuitry: An internal current is generated that is modulated by the electrical field. The EPIC implements (together with the EPS) a non-contact electrometer, in the sense that there is no direct DC path from the outside world to the sensor input; a condition that is somewhat analogous to the gate electrode of a MOS transistor. The electrode is protected by a capping layer of dielectric material to ensure that the electrode is electrically insulated from the body being measured. The device is AC coupled, e.g. with a lower corner frequency (-3 dB) of a few tens of MHz and an upper corner frequency above 200 MHz. This frequency response is adjustable and can be tailored to suit a particular application. Such an electrometer cannot be DC coupled because the Earth's electric field close to the surface is ≈100-150 V/m.

In single-ended mode, the device can be used to read an electric potential. When used in differential mode, it can measure the local electric field; or it can be deployed in arrays to provide spatial potential mapping (locating a conducting or dielectric material placed within free space).

FIG. 1 shows a basic block diagram of an exemplary EPIC sensor (cf. e.g. [Prance et al., 1997], or [Harland et al.; 2002]). The size of the electrode is somewhat arbitrary and depends on the input capacitance required for a particular application. For bodies placed close to the electrode, the electrode's size is important and the device operation can be understood in terms of capacitive coupling. For devices that are several meters away, the coupling capacitance is defined only by the self-capacitance of the electrode and the device's response is largely a function of the input impedance as it interacts with the field. This is rather counterintuitive but is a function of the very small amount of energy that EPIC takes from the field in active mode.

FIG. 2 shows an embodiment of an electric potential sensor. Figure 2 shows one embodiment of an electric potential sensor. The material is typically a solid metal (e.g. copper) that is covered by an insulating material.
See further at http://www.plesseysemiconductors.com/epic-plessey-semiconductors.php

There is a number of patents covering the EPIC technology; 602 32 911.6-08 (DE); AU2007228660; CA2646411; CN200780026584.8; EP1451595 (CH); EP1451595 (ES); EP1451595 (FR); EP1451595 (IE); EP1451595 (IT); EP1451595 (NL); EP2002273; EP2047284; EP2174416; GB1118970.1; JP2009-500908; JP4391823; TW097126903; TW1308066; US12/293872; US12/374359; US12/669615; US13/020890; US13/163988; US7885700.

### Acoustic beamformers and remote microphones

Multi-microphone arrays, and the associated signal processing, have made great strides towards solving the acoustic scene analysis problem. With the right configuration of microphones and sufficient guiding information, excellent localization and separation can often be achieved. The most serious obstacle is the lack of a simple way to steer the selective acoustic processing towards the desired source within a scene. Selecting the wrong source for enhancement can obviously lead to an unwanted outcome.

Another important solution for hearing impaired subjects is to use a remote microphone close to the target to improve the signal-to-noise ratio (SNR). In a situation with multi-remote microphones the problem arise how to choose (steer) the most important remote microphone (or combination of remote microphones).

Attentional state via eye-tracking using infrared optical sensors as well as eye- and head-position monitoring (using accelerometers, magnetometers and gyroscopes) to steer a beamformer has shown positive results for hearing impaired (cf. e.g. [Kidd et al.; 2013]).

### Electroocculography (EOG)

The EOG signal represents an electrical measure of the eye position that is measured as a voltage difference between electrodes placed typically on either side of the head near the eyes. This is possible due to the permanent front-to-back electrical polarization inherent to the physiology of the eyes (cf. e.g. [Carpenter; 1988]).

In an earlier patent application (US2014369537A1), the present inventors have proposed to provide eye gaze control via Electroocculography from the ear canal (EarEOG). Electroocculography senses the eye position electrically. This topic is also dealt with in [Manabe & Fukamoto; 2010] and in US20140198936A1.

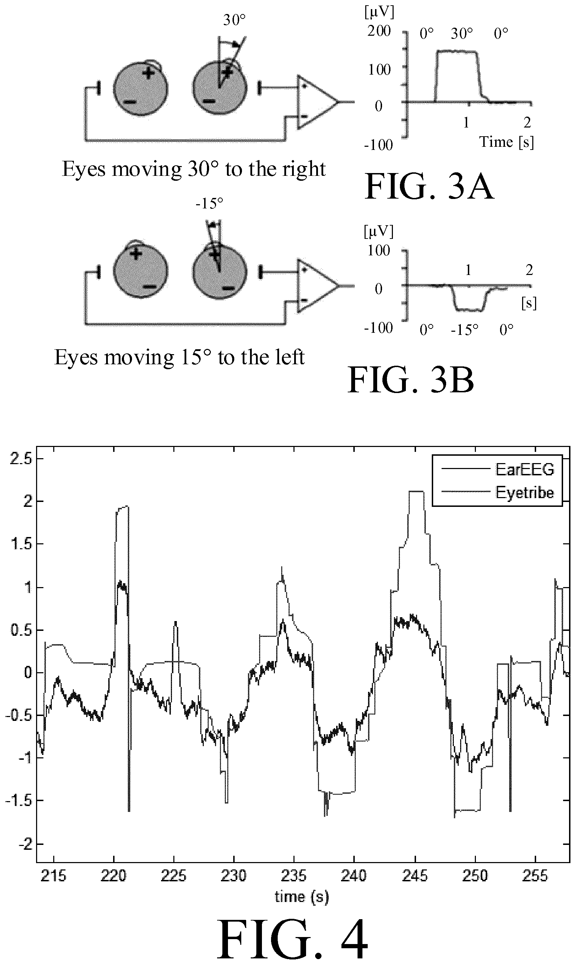

FIG. 3 shows the use of electrooculography (EOG) to detect eye movement, FIG. 3A showing an EOG signal detecting an eye-movement of the eyes to the right, FIG. 3B showing an EOG signal detecting an eye-movement to the left.

FIG. 4 shows EarEEG signals picked up in the left and right ears and subtracted from each other and post processed to remove a DC component to create the EarEOG signal (curve, labelled earEEG), and this signal is compared to an eye-tracker signal based on infrared tracking of the eye gaze (curve, labelled EyeTribe). The problem for the EarEOG solution in hearing aids is that there is a need for an electrical wire to connect the left and right electrodes.

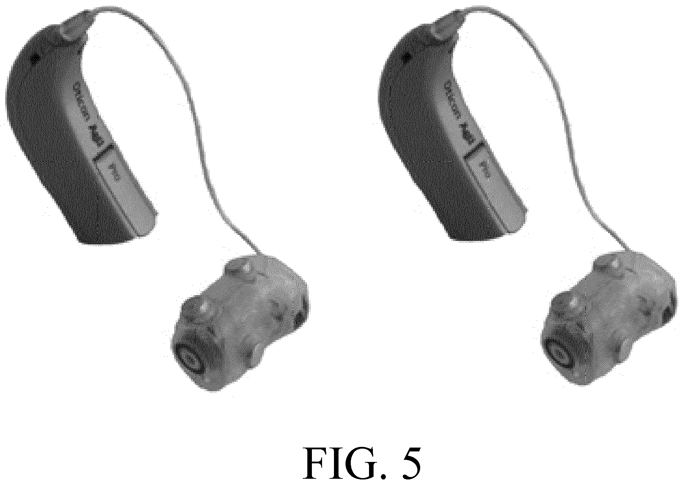

FIG. 5 shows a pair of behind the ear (BTE) hearing devices with EEG electrodes on the mould surface. A solution to the above problems with skin contact and need for a wire between the ears is to use EPS sensor(s) with integrated circuits (EPIC) in the ear canal (instead of conventional electrodes, as shown in FIG. 5) in either one ear or both ears. The solutions can be made possible in e.g. in-the-ear moulds, domes or integrated in a behind the ear solution. In an embodiment, the EPS comprise 'conventional electrodes' for establishing a direct electric contact between skin and electrode.

[Hart et al., 2009] describes selection of an auditory source using an eye tracker (camera and processing capacity) for monitoring a user's eye movement.

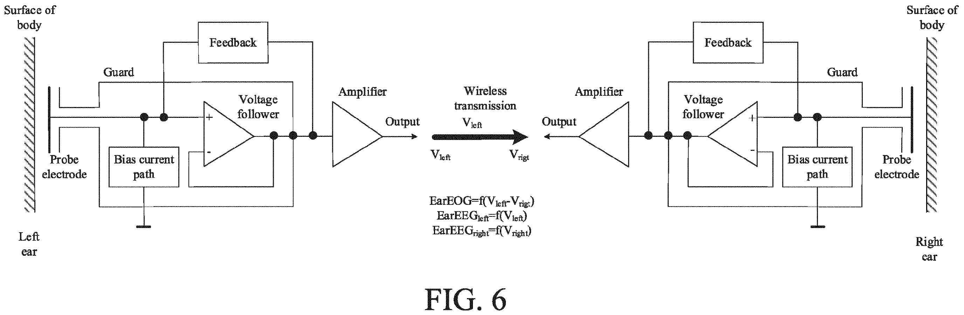

FIG. 6 shows illustrates an EPS electrode in left and right ears (e.g. included in left and right ear pieces (e.g. hearing aids), located at left and right ears, respectively, or fully or partially in left and right ear canals of the user, respectively), respectively, wherein the EarEEG signals picked up at one EPS electrode at one ear is transmitted to the other ear to create a difference signal, whereby EOG in the ear, EarEOG, is provided. At a given point in time, a) the left and right EPS sense potentials P_{left} and P_{right}, respectively, b) compare the sensed potentials to a reference potential (e.g. a virtual ground P₀, cf. e.g. EP2997893A1), and c) provide respective amplified voltages V_{left} = A(P_{left} - P₀) and V_{right} = A(P_{right} - P₀), where A is an amplification factor. The left and right un-amplified or amplified voltages V_{left} and V_{right}, may (in an EEG measurement mode) be representative of respective left and right EEG signals, (due to the location of the electrodes) termed EarEEG_{left} and EarEEG_{right}, respectively. In other words, the left and right EarEEG signals are functions (f) of left and right voltages picked up by the left and right EPS, EarEEG_{left} = f(V_{left}) and EarEEG_{right} = f(V_{right}). In an eye gaze detection mode (where eye gaze is 'provided' by the user), the measured potentials are representative of eye movement, and by transmitting one of the left and right amplified voltages V_{left} and V_{right} to the respective other ear piece (or to another device) or exchange said amplified voltages between the left and right ear pieces (or transmitting said amplified voltages to another device, e.g. a processing device), an EarEOG signal representative of eye gaze can be determined based on the left and right amplified voltages V_{left} and V_{right}. The EarEOG signal is a function (f) of a difference between the left and right amplified voltages V_{left} and V_{right}, EarEOG=f(V_{left} - V_{right}). The function (f) for EarEEG and for EarEOG may in an embodiment be different. The transmission of left and/or right amplified voltages V_{left} and V_{right} to another device may be performed via a wired or wireless link. The wireless link may be based on radiated fields (e.g. based on a proprietary or standardized protocol, e.g. Bluetooth, such as Bluetooth Low Energy) or based on near-field communication, (e.g. based on an inductive coupling between respective coil antennas, and based on a proprietary or standardized protocol).

### Robust eye-gaze assessment using EAREOG

A real-time eye-gaze model can be developed based on the input from one or several EarEEG electrodes (based on EPS-electrodes, e.g. on 'conventional electrodes' for establishing a direct electric contact between skin and electrode) from each ear to form a robust EarEOG control signal. The EarEOG signal is highly correlated with the real eye-gaze direction relative to the forward (nose pointing) direction (cf. FIG. 4). The eye-gaze model can be based on control theory (cf. e.g. [Ljung; 1999]) and include artefact rejection to make the model robust against eye-blinks and muscle artefacts, as well as drift of electrodes and eventually varying ambient light conditions (cf. e.g. [Berg & Scherg; 1991]).

### Absolute gaze angle assessment using EAREOG and headtrackers

A real-time model of absolute gaze angle (looking direction) in space can be developed. The absolute gaze angle model can take the EarEOG control signal as input as well as 9 dimensional headtrackers (3D accelerometer, 3D magnetometer, 3D gyroscope) to real-time calibrate the eye gaze angle in absolute room coordinates. Movement data can thereby be used to increase the reliability of the eye-position estimates; the obligatory ocular counter rotation (nystagmus) during head movement [Carpenter; 1988]. By detecting these such movement and correlating eye to head movement this source of information can be used to continually adjust and recalibrate the angle derived from the EarEOG signal. The combination of EOG (e.g. EarEOG) signals (providing eye gaze relative to the head, e.g. to the direction of the nose) and information from a 9 dimensional head tracker (providing a position of the head) can be seen as equivalent to an eye camera.

### Example 1: Wireless remote microphone steering

A setup can be implemented where wireless remote microphones are placed at e.g. 4-5 spatially separated directions worn on or close persons with which the wearer of the hearing device(s) is intended to communicate, for example in a restaurant situation. The absolute gaze angle can be used to steer the emphasis of the remote microphone in the gaze direction.

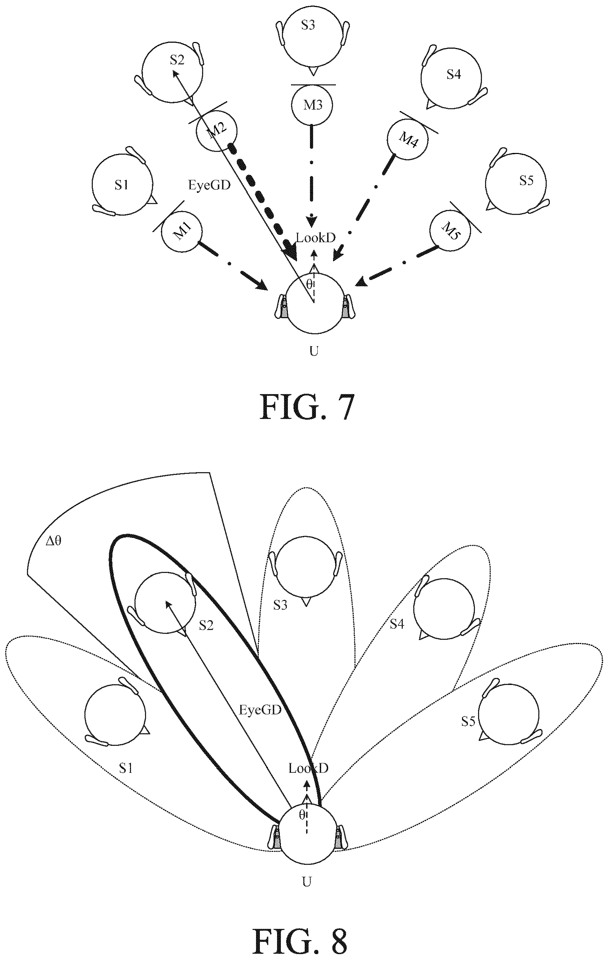

FIG. 7 shows an application scenario of a hearing device or a pair of hearing devices according to the present disclosure using EarEOG in a situation with multiple talkers (S1, S2, S3, S4, S5). N remote microphones (M1, M2, M3, M4, M5, N=5 in the figure) are wirelessly sending their signals to the hearing aids (as indicated by arrowed lines from the microphone units towards the user). From the head position of the user (U), absolute angles can be determined, which separate the horizontal space into a number of sections where remote microphone M1, M2, M3, M4 is expected to be located. In an embodiment, The EarEOG signal is used to control which of the wirelessly received signals from the N microphones to present to the wearer of the hearing device(s) (i.e. to control a selection of input signal among the N available input signals). In FIG. 7, the 2^{nd} talker S2 is selected (via eye gaze) and the hearing aid is consequently configured to provide the signal received from the second microphone (M2) and present the corresponding signal to the user (U) via an output transducer (e.g. a loudspeaker) of the hearing device. This is indicated in FIG. 7 by the eye gaze direction EyeGD pointing towards the 2^{nd} speaker (S2) (corresponding to eye gaze angle θ), and the second wireless link being shown as a boldface dotted arrow from the 2^{nd} speaker (S2) towards the user (U).

Another complementing solution would be to use visually based eye-trackers from e.g. glasses with cameras (http://www.eyetracking-glasses.com/; http://www.tobiipro.com/product-listing/tobii-pro-glasses-2/) or Eye-glasses with EOG (see e.g. Jins Meme, https://jins-meme.com/en/eyewear-apps/).

### Example2 - Steering a beamformer

FIG. 8 illustrates a situation of use EarEOG control of a beamformer of a hearing device according to the present disclosure. Steerable real-time beamformers can implemented in hearing devices. In an embodiment, an EarEOG control signal is used to steer the beamformer angle of maximum sensitivity towards the gaze direction.

The scenario of FIG. 8 illustrates a table situation with quasi constant location of a multitude of occasional talkers/listeners (S1-S5). In an embodiment, the hearing devices of a hearing system according to the present disclosure provides eye gaze control (i.e. comprise electrodes for picking up body potentials and adapted for exchanging amplified voltages based thereon to provide a control signal representative of a current eye gaze direction (EyeGD in FIG-. 8) of the user, e.g. relative to a look direction (LookD in FIG. 8) of the user). Each hearing device comprises a beamformer for providing a beam (i.e. the microphone system has a maximum in sensitivity in a specific direction) directed towards a target sound source, here a talker in the vicinity of the user, controlled by a user's eye gaze. For such situation, predefined look vectors (transfer functions from sound source to microphones) and/or filter weights corresponding to a particular direction may be determined and stored in a memory unit MEM of the hearing device, so that they can be quickly loaded into the beamformer, when a given talker is selected. Thereby the non-active beams can be considered to represent virtual microphones that can be activated one at a time.

FIG. 8 shows an application of a hearing system according to the present disclosure for segregating individual sound sources in a multi-sound source environment. In FIG. 8, the sound sources are persons (that at a given time are talkers (S) or listeners (L)) located around a user (U, that at the time illustrated is a listener (L)). The user (U) wears a hearing system according to the present disclosure that allows segregation of each talker and allows the user to tune in depending on the person (S1, S2, S3, S4, S5) that is currently speaking as indicated by (schematic) elliptic beams of angular width (Δθ) sufficiently small to enclose (mainly) one of the persons surrounding the user. In the example of FIG. 8, the person speaking is indicated by S2, and the sound system is focused on this person as indicated by direction θ of eye gaze of the user (EyeGD) and a bold elliptic beam including the speaker (S2).

In an embodiment, the hearing device or devices of the hearing system worn by the user (U) are hearing devices according to the present disclosure. Preferably, the hearing system comprises two hearing devices forming part of a binaural hearing system, e.g. a binaural hearing aid system. In an embodiment, the sensor part of the hearing devices comprises a number of electromagnetic sensors each comprising a sensing electrode configured to be coupled to the surface of the user's head (e.g. at or around an ear or in an ear canal), when the hearing device is operatively mounted on the user. In an embodiment, the sensor part comprises an electrical potential sensor for sensing an electrical potential. In another embodiment, the sensor part comprises a magnetic field sensor for sensing a magnetic field (e.g. generated by a user's body, e.g. originating from neural activity in the user's head, e.g. the brain). In an embodiment, the electrical potential and/or magnetic field sensors are configured to sense electric and/or magnetic brain wave signals, respectively. In an embodiment, the sensing electrode(s) is(are) configured to be capacitively or inductively coupled to the surface of the user's head, when the hearing device is operatively mounted on the user. In an embodiment, the electrical potential sensor comprises a sensing electrode configured to be coupled to the surface of the user's head (e.g. at or around an ear or in an ear canal), when the hearing device is operatively mounted on the user. In an embodiment, the sensing electrode is configured to be directly (e.g. electrically (galvanically)) coupled to the surface of the user's head (e.g. via a 'dry' or 'wet' contact area between the skin of the user and the (electrically conducting) sensing electrode), when the hearing device is operatively mounted on the user.

Another complementing solution would be to use visually based eye-trackers from e.g. glasses with cameras (http://www.eyetracking-glasses.com/) or Eye-glasses with EOG (see e.g. Jins Meme, https://jins-meme.com/en/eyewear-apps/).

In a scene with visual and auditory objects, e.g. like the restaurant problem solver (RPS) scenario (see FIG. 7, 8), there are a number of interesting objects (e.g. three) with respect to which control of the acoustic input would be needed. Control of acoustic input may for example comprise one or more of (1) controlling the blending mixture of remote microphones placed close to the object's mouth, (2) controlling one or several multi-microphone beamformers in a headworn device (cf. FIG. 8), or (3) using distributed microphone networks (cf. FIG. 7).

By Kalman-filtering the output from (Ear)EOG sensors (or other eye trackers) the eye-angle (cf. e.g. angle θ in FIG. 8) relative to the head's nose-pointing direction (cf. e.g. LookD in FIG. 8) can be estimated. By Kalman-filtering the output from 9DOF sensors (9 degree of freedom sensors, 3D accelerometer, 3D gyroscope, 3D magnetometer), or other motion-tracking devices - placed at head level close to the ear - the *absolute* head-angle relative to the room can be determined. By combining the outputs from the (Ear)EOG and 9DOF another (or the same) Kalman filter can be made whose output is the absolute eye-angle relative to the room.

By further Kalman-filtering (e.g. using another or the same Kalman filter) the output from the absolute eye-angle relative to the room for Simultaneous Location and Mapping (SLAM), a kind of 'hotspot(s)' can be estimated, where some eye-gaze angles are more plausible than others (the person is probably looking more at the persons in the scene than at the backgrounds). The principle idea is to extend the Kalman filter, where eye-gaze angle is a state, with a number of states/parameters that describe the angle to the 'hotspots' (the Map in general robotic-terms). This principle works well if you switch between a number of discrete hotspots as the case is in this application. The Map can be points or normal-distributions, assuming that the eye-gaze angle follow a mix of gauss-distributions.

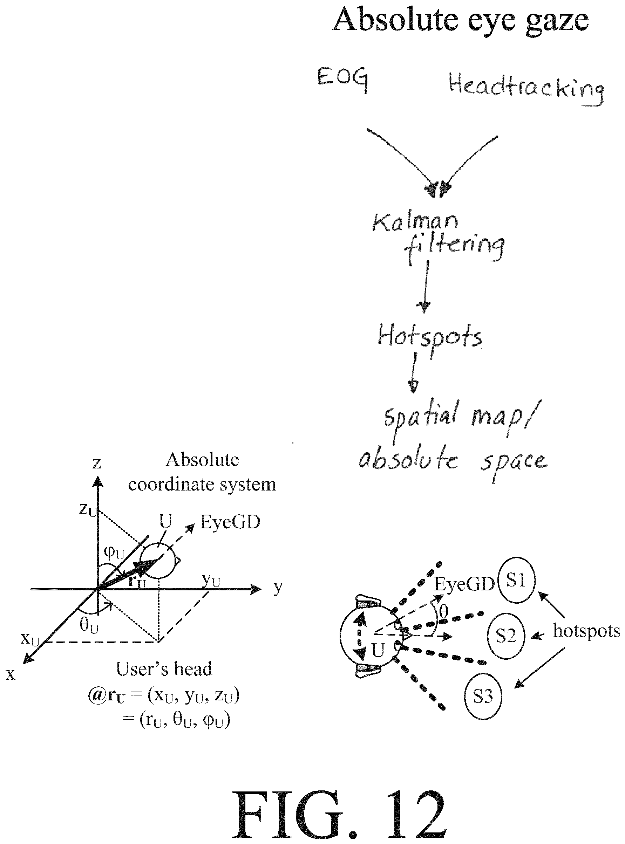

The above procedure is illustrated in FIG. 12. FIG. 12 schematically illustrates a method of providing data for controlling functionality in a head worn hearing device, e.g. a hearing aid, by a combination of EarEOG data (*EOG* in FIG. 12), as described in connection with FIG. 2A, 3B, 4, 5, 6, 7, 8) with head tracking data (*Headtracking* in FIG. 12). The ear EOG measurements provide a direction (EyeGD) relative to a user based on eye gaze extracted from sensors (electrodes, cf. e.g. FIG. 5) located at the ear of the user. The head tracking data provides absolute coordinates (in a Cartesian or spherical coordinate system, cf. *Absolute coordinate system* in the lower left part of FIG. 12) of the head of the user (U) at a given location (xu, yu, zu) or in spherical coordinates (r_{∪}, θ_{∪}, ϕ_{∪}). The centre (origo) of the coordinate system can be at any appropriate location, e.g. a center or corner of a room, or at a center of a GPS coordinate system. By combining these data, absolute eye gaze angles in a fixed coordinate system can be determined (e.g. in a two dimensional (e.g. horizontal plane)). These data may e.g. be further improved by Kalman filtering (cf. *Kalman filtering* in FIG. 12), e.g. to reduce drift (cf. e.g. [Manabe & Fukamoto; 2010]). Based thereon, the locations of hotspots (in preferred eye gaze directions of the user), cf. sound sources S1, S2, S3 in the lower right part of FIG. 12, can be determined in absolute or relative coordinates. The hotspots (cf. 'Hotspot' in FIG. 12) may be determined in absolute coordinates and thus represent a spatial map of 'acoustically interesting locations' for the user (U) (cf. *'spatial map*/*absolute space'* in FIG. 12). For a hearing aid application, locations of preferred sound sources (hotspots) *relative* to the user (specifically to the hearing aids) would be of interest to simply calculations (and thus reduce power consumption), e.g. in connection with beamforming or selection of active wireless links (cf. e.g. FIG. 7, 8).

### Kalman filtering for EarEOG (1)

One embodiment of Kalman estimation of eye-gaze angle relative to the head is found in the model defined in Section 3.2.1 of [Komogortsev & Khan; 2009], using the (Ear)EOG signal as the eye-tracker signal. A simplified version using only position and speed as states can also be seen as an embodiment.

### Kalman filtering for Head angle (2)

One embodiment of the estimation of the absolute head angle relative to the room using head-mounted 9DOF sensors can be found in the "Statistical Sensor Fusion - Lab 2, Orientation Estimation using Smartphone Sensors", by representing Rotations using Quaternions, see section 3.1 in the document. Another source of information is [Kuipers; 2000], where more equations for "Quaternions and Rotation sequences" can be found.

### Hotspots - Kalman filtering for simultaneous location and mapping (SLAM) (3)

An introduction to Simultaneous Location And Mapping (SLAM) can e.g. be found in the tutorial by [Bailey & Durrant-Whyte; 2006]. Various SLAM algorithms are implemented in the open-source robot operating system (ROS) libraries. Mapping (SLAM) describes the computational problem of constructing or updating a map of an unknown environment while simultaneously keeping track of an agent's location within it. While this initially appears to be a chicken-and-egg problem, there are several algorithms known for solving it, at least approximately, in tractable time for certain environments. Popular approximate solution methods include the particle filter and extended Kalman filter.

In the present context, it is proposed to extend the Kalman filter, where eye-gaze angle is a state, with a number of states/parameters that describe the angle to the 'hotspots' (the Map in general robotic-terms). This principle works well if you switch between a number of discrete hotspots as the case is in this application. The Map can be points or normal-distributions, assuming that the eye-gaze angle follow a mix of gauss-distributions.

One embodiment can be separate Kalman filters for separate stages, cf. (1)-(3) above. Another embodiment can be one Kalman filter solving the whole problem.

The Kalman filter can in simple tracking models be replaced by the recursive least squares (RLS, Recursive Least Squares), the least mean square (LMS, Least Mean Squares), or any other adaptive algorithm.

FIG. 16 illustrates the use of a Kalman filter (KF) together with a change detector in tracking models. The Kalman filter (KF) can be supplemented by a CUSUM detector (CUSUM, CUSUM=CUmulative SUM), whose alarm is fed back to the KF to enable an instantaneous increase in tracking speed. In this way, the KF state estimate can have both good noise attenuation and occasional fast tracking speed at the same time. The role of the change detector is to detect fast changes in the gaze angle, and then instantaneously increase the tracking speed. This is a way to circumvent the inherent trade-off in any linear filter, including KF, RLS and LMS, that fast tracking speed and high accuracy (good noise attenuation) cannot be achieved at the same time. However, a linear adaptive filter with a nonlinear change detector can provide a leap in performance. In one embodiment, the CUSUM detector can be used, and when an alarm is issued, the covariance matrix in the Kalman filter is artificially increased. It can be shown that an adaptive filter can only lowpass filter gaze data, while the KF-CUSUM combination can find the fixation points with high accuracy. Change detection using a combination of Kalman filtering and CUSOM is e.g. described in [Severo & Gama; 2010]. The mathematical principles involved in CUSUM is e.g. described in https://en.wikipedia.org/wiki/CUSUM.

In the block diagram, the Kalman filter (KF) takes as inputs the sensor observation yₜ and optional external signals ut (e.g. from accelerometers, gyroscopes, magnetometers, etc.). The KF estimates a state *x̂*_{t|*t-*1} and its covariance matrix Pₜ, together with sensor signal prediction errors εₜ. A change detector can operate on a subset of these, in the simplest case just testing the whiteness of the residuals (are they independent over time, is the variance or the whole distribution invariant over time). When a test statistic exceeds a threshold, an alarm (Alarm in FIG. 16) is issued. In this case, it means that the Kalman filter (KF) is not operating under its assumptions, where one cause might be a sudden change in the state vector not included in the model. One remedy to this is to momentarily increase the covariance Pₜ in one way or another (there are multiple, well known solutions in the literature on Kalman filters how to blow up the covariance).

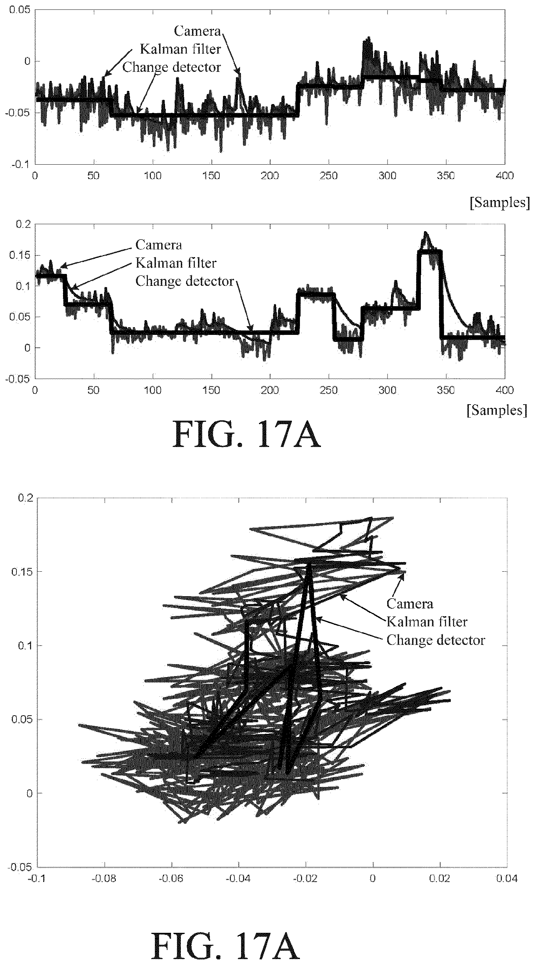

FIG. 17A, 17B show the gaze direction as obtained from a camera. But these data have much in common with the differential EEG signal. 17A shows gaze up-down (top) and left-right (bottom) (in normalized length or angle units) as a function of time (400 samples). FIG. 17B illustrates the data of FIG. 17A in a pan-tilt plane to illustrate the two dimensional points of fixation. What we can see from both figures is that the gaze apparently is attracted by a few fix points. In the experiment, it is a car driver who is watching the traffic in front of the car, but occasionally looks in the rear mirrors and the dashboard. The goal is the same as for the hearing aid, to monitor which fix point the user is looking to and take measures to assist him.

Another estimator of the angle to an object of interest (e.g. an audio source) is to use direction-of-arrival detectors from the acoustic input, cf. e.g. EP3013070A2.

### Example 3 - Individually made electrodes

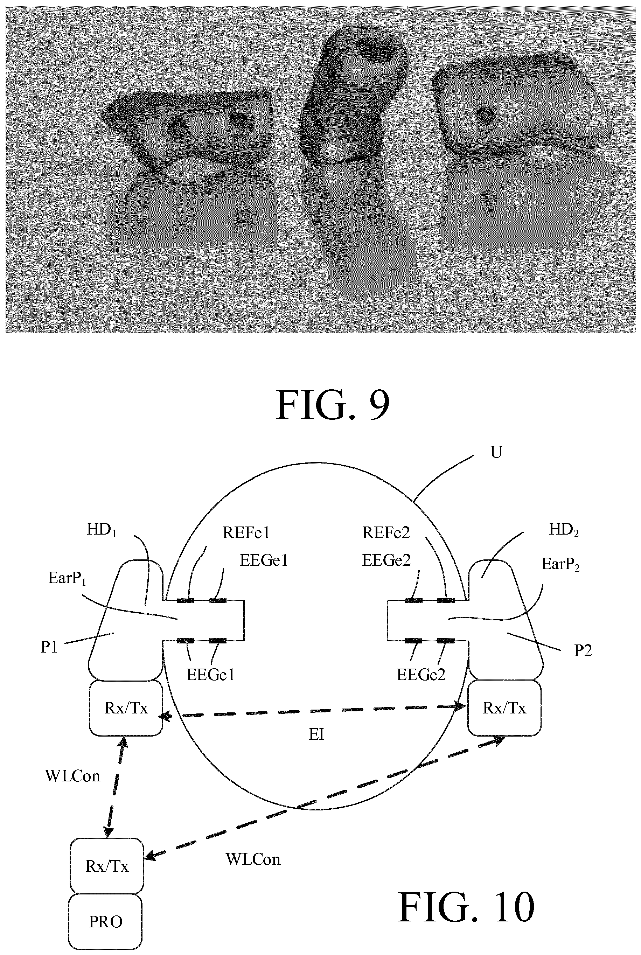

FIG. 9 shows an example of individually 3D-printed ear mould made in stainless steel. The solid metal in the EPS may be made by individually printed forms of the ear canals.

### Example 5 - Mouse control of a computer / smartphone

In a further use scenario, the EarEOG signal (with or without head tracking data) is sent to a computer smartphone to control a mouse on a screen. In this case there is need for two EPS electrodes or two active EarEEG electrodes per ear. The two (or more) electrodes within each hearing aid are aligned in the vertical direction to capture up/down eye deflections. The horizontal EarEOG signal is generated as described above.

Detection of blinks (large artefacts having voltages above 80-300 micro volt) can be used as mouse-clicks.

### Example 6 - Control for e.g. paralysed persons

The mouse control via EarEOG of a screen can be used to steer e.g. dedicated programs for the paralysed, cf. e.g. https://powermore.dell.com/business/eye-tracking-technology-allows-paralyzed-people-to-control-pcs-tablets/.

FIG. 10 shows a use scenario of an embodiment of a hearing assistance system comprising EEG and reference electrodes according to the present disclosure in the form of electrical potential sensors.

In the embodiment of FIG. 10, the first and second hearing devices (*HD₁, HD₂*) comprises first and second parts (*P1, P2*)*,* respectively, adapted for being located at or in an ear of a user (U). Further, the first and second hearing devices (*HD₁, HD₂*) each comprises EEG-electrodes *(EEGel, EEGe2)* and a reference electrode (*REFe1, REFe2*)*,* respectively, arranged on the outer surface of the respective ear pieces *(EarP1, EarP₂).* The ear pieces are each being adapted to be located at the ear or fully or partially in the ear canal. When the first and second hearing devices are operationally mounted on the user, the electrodes of the ear pieces are positioned to have electrical contact with the skin of the user to enable the sensing of brainwave signals. The ear pieces *EarP1, EarP₂* constitute or form part of the first and second parts *P1, P2.* Each of the first and second parts (*P1, P2*) comprises a number of EEG-electrodes (*EEGe1, EEGe2*)*,* here 3 are shown (but more or less may be present in practice depending on the application), and a reference electrode (*REFe1, REFe2*)*.* Thereby the reference voltage (V_{REF2}) picked up by the reference electrode (*REFe2*) of the second part (*P2*) can be used as a reference voltage for the EEG potentials (V_{EEGli}) picked up by the EEG electrodes *(EEGel)* of the first part (*P1*)*,* and vice versa. In an embodiment, the first and second hearing devices provides a binaural hearing assistance system. The reference voltages (V_{REF1}, V_{REF2}) may be transmitted from one part to the other (P1 <-> P2) via electric interface *EI* (and optionally via an auxiliary device *PRO,* e.g. a remote control device, e.g. a smartphone). The auxiliary device (*PRO*) may e.g. be configured to process EEG-signals (and optionally performing other processing tasks related to the hearing assistance system) and/or providing a user interface for the hearing assistance system. Each of the first and second hearing devices (*HD₁, HD₂*) and the auxiliary device (*PRO*) comprises antenna and transceiver circuitry (*Rx*/*Tx*) configured to establish a wireless link (*WLCon*) to each other. The two sets of EEG-signal voltage differences (*ΔV_{EEG1}, ΔV_{EEG2}*) can be used separately in each of the respective first and second hearing devices (*HD₁, HD₂*) (e.g. to control processing of an input audio signal, e.g. as outlined in the above examples) or combined in one of the hearing devices and/or in the auxiliary device (*PRO,* e.g. for display and/or further processing), e.g. to provide (ear) EOG signals (as discussed in connection with FIG. 6).

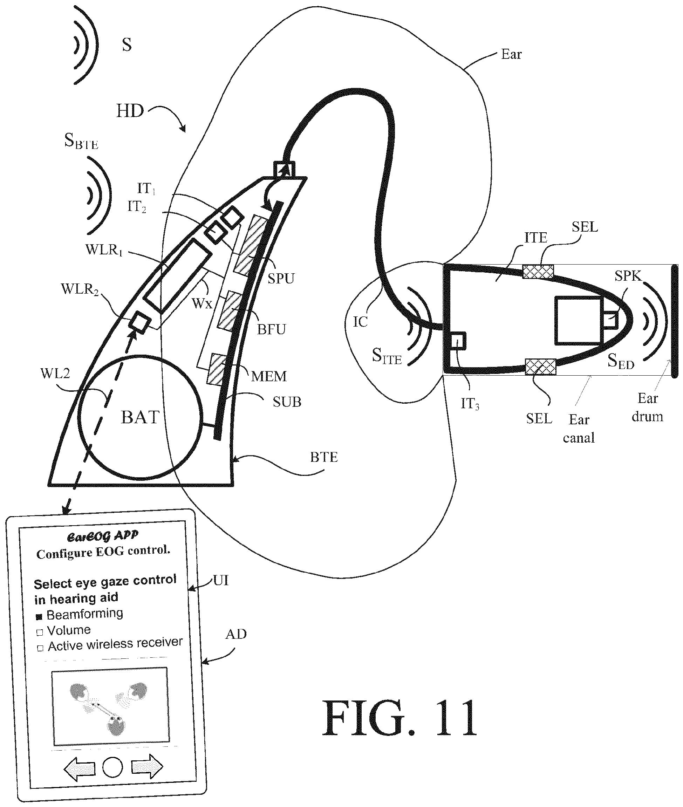

FIG. 11 shows an embodiment of a hearing system comprising a hearing device and an auxiliary device in communication with each other. FIG. 11 shows an embodiment of a hearing aid according to the present disclosure comprising a BTE-part located behind an ear or a user and an ITE part located in an ear canal of the user.

FIG. 11 illustrates an exemplary hearing aid *(HD)* comprising a BTE-part (*BTE*) adapted for being located behind pinna and an part (*ITE*) comprising a housing accommodating one or more sensing electrodes (*SEL,* and possibly associated electric circuitry for generating a corresponding sensing voltage) for capturing electric potentials of the body. The ITE-part may as shown in FIG. 11 further comprise an output transducer (e.g. a loudspeaker/receiver, SPK) adapted for being located in an ear canal (*Ear canal*) of the user and to provide an acoustic signal (providing, or contributing to, acoustic signal *S_{ED}* at the ear drum (*Ear drum*)). In the latter case, a so-called receiver-in-the-ear (RITE) type hearing aid is provided. The BTE-part (*BTE*) and the ITE-part (*ITE*) are connected (e.g. electrically connected) by a connecting element (*IC*)*,* e.g. comprising a number of electric conductors. The BTE part (*BTE*) comprises two input transducers (e.g. microphones) (*IT₁, IT₂*) each for providing an electric input audio signal representative of an input sound signal *(S_{BTE})* from the environment. In the scenario of FIG. 11, the input sound signal *S_{BTE}* includes a contribution from sound source S. The hearing aid *(HD)* of FIG. 11 further comprises two wireless transceivers (*WLR₁, WLR₂*) for transmitting and/or receiving respective audio and/or information signals and/or control signals (including potentials or voltages provided by the sensing electrodes (SEL)). The hearing aid *(HD)* further comprises a substrate (*SUB*) whereon a number of electronic components are mounted, functionally partitioned according to the application in question (analogue, digital, passive components, etc.), but including a configurable signal processing unit (*SPU*)*,* a beam former filtering unit (*BFU*)*,* and a memory unit (*MEM*) coupled to each other and to input and output transducers via electrical conductors Wx. The mentioned functional units (as well as other components) may be partitioned in circuits and components according to the application in question (e.g. with a view to size, power consumption, analogue vs. digital processing, etc.), e.g. integrated in one or more integrated circuits, or as a combination of one or more integrated circuits and one or more separate electronic components (e.g. inductor, capacitor, etc.). The configurable signal processing unit (*SPU*) provides a processed audio signal, which is intended to be presented to a user. In the embodiment of a hearing aid device in FIG. 11, the ITE part (*ITE*) comprises an input transducer (e.g. a microphone) *(IT₃)* for providing an electric input audio signal representative of an input sound signal *S_{ITE}* from the environment (including from sound source S) at or in the ear canal. In another embodiment, the hearing aid may comprise *only* the BTE-microphones (*IT₁, IT₂*)*.* In another embodiment, the hearing aid may comprise *only* the ITE-microphone *(IT₃).* In yet another embodiment, the hearing aid may comprise an input unit *(IT₄)* located elsewhere than at the ear canal in combination with one or more input units located in the BTE-part and/or the ITE-part. The ITE-part may further comprise a guiding element, e.g. a dome or equivalent, for guiding and positioning the ITE-part in the ear canal of the user.

The hearing aid *(HD)* exemplified in FIG. 11 is a portable device and further comprises a battery (*BAT*) for energizing electronic components of the BTE- and possibly of the ITE-parts.

The hearing aid *(HD)* may (as shown) e.g. comprise a directional microphone system (including beamformer filtering unit (*BFU*)) adapted to spatially filter out a target acoustic source among a multitude of acoustic sources in the local environment of the user wearing the hearing aid. The beamformer filtering unit (*BFU*) may receive as inputs the respective electric signals from input transducers *IT₁, IT₂, IT₃* (and possibly *IT₄)* (or any combination thereof) and generate a beamformed signal based thereon. In an embodiment, the directional system is adapted to detect (such as adaptively detect) from which direction a particular part of the microphone signal (e.g. a target part and/or a noise part) originates. In an embodiment, the beam former filtering unit is adapted to receive inputs from a user interface (e.g. a remote control or a smartphone) regarding the present target direction. In an embodiment, the beamformer filtering unit (*BFU*) is controlled or influenced by signals from the sensing electrodes (or processed versions thereof, e.g. EOG-signals representative of eye gaze of the user). In an embodiment, the direction of a beam (or a 'zero point) of the beamformer filtering unit is thereby controlled or influenced. In another embodiment, the input from one of the wireless receivers is selected based on signals from the sensing electrodes (or processed versions thereof, e.g. EOG-signals representative of eye gaze of the user). The memory unit (*MEM*) may e.g. comprise predefined (or adaptively determined) complex, frequency dependent constants (Wᵢⱼ) defining predefined (or adaptively determined) or 'fixed' beam patterns (e.g. omni-directional, target cancelling, pointing in a number of specific directions relative to the user (cf. e.g. FIG. 7, 8), etc.), together defining the beamformed signal Y_{BF}.

The hearing aid of FIG. 11 may constitute or form part of a hearing aid and/or a binaural hearing aid system according to the present disclosure. The processing of an audio signal in a forward path of the hearing aid (the forward path including the input transducer(s), the beamformer filtering unit, the signal processing unit, and the output transducer) may e.g. be performed fully or partially in the time-frequency domain. Likewise, the processing of signals in an analysis or control path of the hearing aid may be fully or partially performed in the time-frequency domain.

The hearing aid (HD) according to the present disclosure may comprise a user interface UI, e.g. as shown in FIG. 11 implemented in an auxiliary device (AUX), e.g. a remote control, e.g. implemented as an APP in a smartphone or other portable (or stationary) electronic device. In the embodiment of FIG. 11, the screen of the user interface (UI) illustrates an *EarEOG APP,* with the subtitle 'Select eye gaze control in hearing aid' (upper part of the screen). Possible functions that can be selected by the user - via the APP - for control via eye gaze are exemplified in the middle part of the screen. The options are 'Beamforming', 'Volume' and 'Active wireless receiver'. In the screen shown in FIG. 11, the option 'Beamforming' has been selected (as indicated by solid symbols ■, and illustrated by the graphical symbol beneath the options). The arrows at the bottom of the screen allow changes to a preceding or a proceeding screen of the APP, and a tab on the circular dot between the two arrows brings up a menu that allows the selection of other APPs or features of the device. In an embodiment, the APP is configured to provide an (possibly graphic) illustration of the currently selected or activated beamformer (cf. e.g. FIG. 15), or volume setting, or wireless connection. The 'Beamforming' and 'Active wireless receiver' may e.g. be controlled by horizontal eye gaze. 'Volume' may e.g. be controlled via vertical eye gaze.

The auxiliary device and the hearing aid are adapted to allow communication of data, including data representative of the currently selected function to be controlled via eye gaze to the hearing aid via a, e.g. wireless, communication link (cf. dashed arrow WL2 in FIG. 11). The communication link WL2 may e.g. be based on far field communication, e.g. Bluetooth or Bluetooth Low Energy (or similar technology), implemented by appropriate antenna and transceiver circuitry in the hearing aid (HD) and the auxiliary device (AUX), indicated by transceiver unit WLR₂ in the hearing aid.

The hearing aid may comprise a number of wireless receivers (e.g. symbolized by WLR₁ in FIG. 11), or may be arranged to receive signals on configurable channels, for receiving different audio signals and/or other signals from a number of transmitters, e.g. from a number of wireless microphones (cf. e.g. FIG. 7). In an embodiment, reception of signals from a given transmitter may be controlled by the user via eye gaze (here derived from EarEOG-signals), cf. mode 'active wireless receiver' of the *EarEOG APP.*

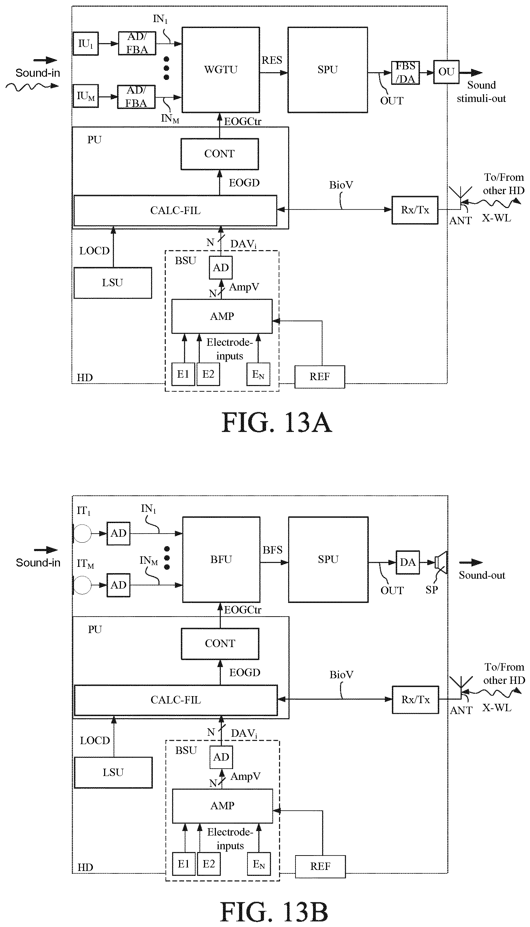

FIG. 13A illustrates a first embodiment of a hearing device according to the present disclosure. The hearing device, e.g. a hearing aid, (HD) comprises a forward path from a number M of input units (IU₁, ..., IU_{M}) for picking up sound or receiving electric signals representing sound ('Sound-in') to an output unit (OU) for providing stimuli ('Sound stimuli-out') representing said sound and perceivable as sound by a user wearing the hearing device. The forward path further comprises a number M of analogue to digital converters (AD) and analysis filter banks (FBA) operationally coupled to each their input unit (IU₁, ..., IU_{M}) and providing respective digitized electric input signals IN₁, ..., IN_{M} in time-frequency representation, each comprising a number K of frequency sub-band signals IN₁(k,m), ..., IN_{M}(k,m), k and m being frequency and time indices, respectively, k=1, ..., K. The forward path further comprises a weighting unit (WGTU) receiving the electric input signals as inputs and providing a resulting signal RES as a weighted combination of the M electric input signals. In other words, RES= IN₁(k,m)*w₁(k,m), ..., IN_{M}(k,m)*W_{M}(k,m), where wᵢ. i=1, ..., M, are real or complex (in general, time and frequency dependent) weights. The forward path further comprises a signal processing unit (SPU) for further processing the resulting signal RES and providing a processed signal OUT. The signal processing unit (SPU) is e.g. configured to apply a level and/or frequency dependent gain or attenuation according to a user's needs (e.g. hearing impairment). The forward path further comprises a synthesis filter bank (FBS) for converting frequency sub-band signals OUT to a single time-domain signal, and optionally a digital to analogue conversion unit (DA) to convert the digital processed time-domain signal to an analogue electric output signal to the output unit (OU).

The hearing device (HD) further comprises a bio signal unit (BSU) for picking up bio signals from the user's body. The bio signal unit (BSU) comprises a sensor part (E₁, E₂, ..., E_{N}) adapted for being located at or in an ear and/or for fully or partially for being implanted in the head of a user. The sensor part comprises an electrical potential sensor for sensing an electrical potential from the body of the user, in particular from the head, e.g. due to brain activity or eye movement. In FIG. 13A and 13B, the sensor part is embodied as electrodes E₁, E₂, ..., E_{N}, which are electrodes of the hearing device configured to contact skin or tissue of the user's head, when the hearing device is operationally mounted on the user (e.g. in an ear canal) or implanted in the head of the user. The bio signal unit (BSU) further comprises an amplifier (AMP), in the form of electronic circuitry coupled to the electrical potential sensor part to provide an amplified output. The amplifier, e.g. a differential amplifier, receives a number of potentials P₁, P₂, ..., P_{N} from the electrodes E₁, E₂, ..., E_{N}, and a reference potential P₀ from a reference electrode (REF), and provides respective amplified voltages V₁, V₂, ..., V_{N}. The amplified voltages V₁, V₂, ..., V_{N} are fed to respective analogue to digital converters (AD) providing digitized amplified voltages DAVᵢ (i=1, .2...., N). In an embodiment, the amplifier (AMP) includes analogue to digital conversion or is constituted by analogue to digital converters.

In an embodiment, at least one (such as all) of the input units comprises an input transducer, e.g. a microphone. In an embodiment, at least one (such as all) of the input units comprises a wireless transceiver, e.g. a wireless receiver, e.g. configured to receive a signal representative of sound picked up by a remote (wireless) microphone.

The hearing device further comprises or is coupled to a location sensor unit (LSU) providing location data (LOCD) representative of a current location of the user, e.g. representative of the user's head, in a fixed coordinate system (e.g. relative to a specific location, e.g. a room). In an embodiment, the location sensor comprises a head tracker. In an embodiment, the location sensor comprises an accelerometer and a gyroscope. In an embodiment, the location sensor comprises a 9 degree of freedom sensor, comprising a 3D accelerometer, a 3D gyroscope, and a 3D magnetometer.

The hearing device further comprises a wireless transceiver (Rx/Tx) and appropriate antenna circuitry allowing reception of bio signals BioV from and transmission of bio signals BioV to a contra lateral hearing device, e.g. amplified voltages V₁, V₂, ..., V_{N}, e.g. eye movement, via a wireless link (X-WL), cf. waved, arrowed line denoted 'To/From other HD' in FIG. 13A and 13B. The bio signals BioV from a contra-lateral hearing device are fed to calculation unit (CALC) and compared to the corresponding locally generated bio signal(s) BioV (e.g. amplified V₁, V₂, ..., V_{N}). In an embodiment, the EarEOG signal is a function (f) of a difference between the left and right amplified voltages V_{left} and V_{right}, EarEOG=f(V_{left} - V_{right}). In an embodiment, each pair of voltages, V_{1,left} and V_{1,right}, ...., V_{N,left} and V_{N,right}, may provide corresponding ear EOG signals, e.g. EarEOG₁= f(V_{1,left} - V_{1,right}), ..., EarEOG₁= f(V_{N,left} - V_{N,right}). In an embodiment, a resulting ear EOG signal at a given time may be found as an average (e.g. a weighted average; e.g. in dependence of the distance of the electrodes in question from the eyes) of the N ear EOG signals.

The hearing aid further comprises a processing unit (PU) for providing a control signal for controlling a function of the hearing device based on the EarEOG signal(s), e.g. selecting wireless reception from a particular person (cf. FIG. 7), or as exemplified in FIG. 8, 13A, and 13B, controlling the beamformer unit (BF), e.g. selecting one of a number of predefined beamformers in dependence of an eye gaze control signal EOGCtr. The predefined beamformers may e.g. be stored in a memory of the hearing device, e.g. as sets of beamformer filtering coefficients, each corresponding to a given one of a number of predefined locations of a sound source of interest. The processing unit (PU) comprises a calculation unit (CALC) configured to combine the location data LOCD with the digitized amplified voltages DAVᵢ (i=1, .2...., N), representative of (ear) EEG and/or (ear) EOG signals, from the (local) bio signal unit (BSU) and received from a bio signal unit (BSU) of a contra-lateral hearing device (cf. e.g. wireless link X-WL in FIG. 13A, 13B), to provide combined location data. The processing unit (PU) further comprises a Kalman filter (FIL) (or one or more Kalman filters) for filtering the combined location data and providing eye gaze angles in a fixed coordinate system, cf. EOG data signal EOGD. The EOG data signal EOGD is forwarded to control unit CONT. Control unit (CONT) provides control signal EOGCtr to the beamformer unit (BFU) based on the EOG data signal EOGD and is configured to select or provide information (e.g. beamformer filtering coefficients) about the current location (or direction) of interest to the user.

In a specific mode of operation (a 'learning mode'), the calculation unit may be configured to determine locations of hotspots representing preferred eye gaze directions of the user based on said combined location data (cf. e.g. 'hotspots' S1, S2, S3 in FIG. 12). The locations (e.g. represented in a fixed coordinate system) may be stored in a memory of the hearing device (or in an auxiliary device, e.g. a smartphone or the like). The locations may e.g. be displayed via a user interface (e.g. via an app of a smartphone), cf. e.g. FIG. 15.

FIG. 13B illustrates a second embodiment of a hearing device according to the present disclosure. The embodiment of FIG. 13B is identical to the embodiment of FIG. 13A, except that the input units (IU₁, ..., IU_{M}) of FIG. 13A are implemented as microphones (IT₁, ..., IT_{M}) in FIG. 13B.

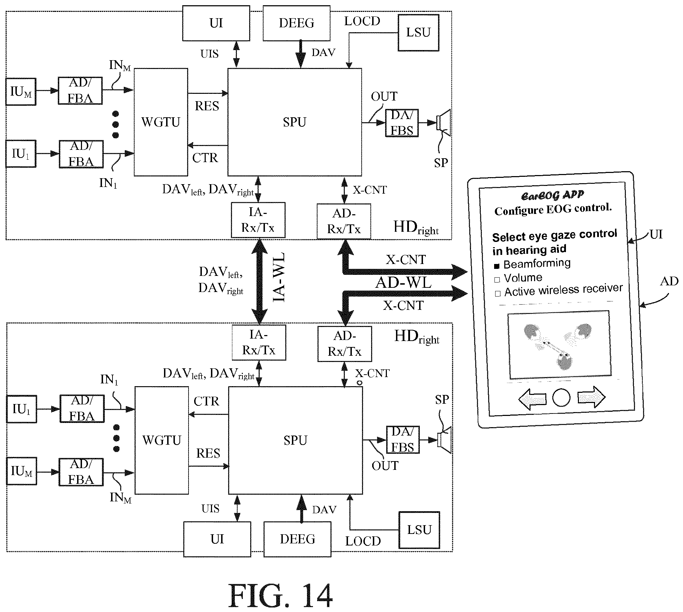

FIG. 14 shows an embodiment of a binaural hearing system comprising left and right hearing devices (HD_{left}, HD_{right}) and an auxiliary device (AD) in communication with each other according to the present disclosure. The left and right hearing devices are adapted for being located at or in left and right ears and/or for fully or partially being implanted in the head at left and right ears of a user. The left and right hearing devices and the auxiliary device (e.g. a separate processing or relaying device, e.g. a smartphone or the like) are configured to allow an exchange of data between them (cf. links IA-WL and AD-WL in FIG. 14), including exchanging the amplified output from electronic circuitry coupled to the electrical potential sensor part (comprising bio sensors, cf. respective units DEEG), or signals based thereon, e.g. EarEEG and/or EarEOG signals, which are fully or partially picked up by the respective left and right hearing devices. The binaural hearing system comprises a user interface (UI) fully or partially implemented in the auxiliary device (AD), e.g. as an APP, cf. *EarEOG* APP screen of the auxiliary device in FIG. 14 (cf. also FIG. 11). In the embodiment, of FIG. 14. The user interface elements (ÙI) on the hearing device(s) may e.g. indicate a communication interface or an (e.g. supplementary or alternative) activation element.

The left and right hearing devices of FIG. 14 may e.g. be implemented as shown in FIG. 13A or 13B. The control of the weighting unit (WGTU) in the embodiment of FIG. 14 is provided by control signal CTR from the signal processing unit (SPU, and may e.g. be based on eye gaze, e.g. via EOG control signal EOGCtr). In the embodiment of FIG 14, the signal processing unit (SPU) is assumed to include (at least some of) the functions of the processing unit (PU) in the embodiments of FIG. 13A and 13B.

FIG. 15 shows a scenario comprising a binaural hearing system comprising left and right hearing devices (*HD_{left},* HD_{right}) and a portable (e.g. handheld) auxiliary device (AD) in communication with each other. The auxiliary device is configured to run an APP implementing a user interface (UI) for a hearing system according to the present disclosure. The auxiliary device (AD) may e.g. constitute or form part of a remote control or a SmartPhone, functioning as a user interface (*UI*) for the hearing system. Each of the first and second hearing devices (*HD_{left}, HD_{right}*) comprises a BTE- and an ITE-part adapted for being located behind and in an ear, respectively of the user, and e.g. electrically connected via a connecting element (cf. e.g. FIG. 11). The first and second ITE-parts and/or the first and second BTE-parts comprise electrodes as discussed in connection with FIG. 1, 5, 6, and 10. The first and second BTE- and/or ITE-parts may further (each) e.g. comprise one or more input transducers, and an output transducer. In an embodiment, the BTE-parts (and the connecting elements) are dispensed with, so that all functionality of the hearing devices (*HD_{left}, HD_{right}*) is located in the respective ITE-parts (*ITEₗ, ITEᵣ*)*.* The first and second BTE-parts may e.g. comprise a battery, one or more input transducers, a signal processing unit and wireless transceivers. In an embodiment, first and second BTE-parts each comprise an output transducer and the attached first and second connecting elements each comprise an acoustic conductor, e.g. a tube, for propagating sound from the output transducer of a BTE-part to the corresponding ITE-part (and thus to the ear drum of the ear in question). The ITE part may comprise a, possibly customized, ear mould. In an embodiment, the hearing assistance system comprises the auxiliary device (*AD* and the user interface *UI*)*.* In an embodiment, the user interface is configured to display information related to the hearing system, e.g. to the identification and analysis of acoustic 'hotspots' (cf. e.g. FIG. 7, 8, and FIG. 12), e.g. an estimate of the multitude of sound sources (here S₁, S₂, S₃) that the user is most likely trying to listen to, and possibly an estimate of their location relative to the user. In the EarEOG control scenario displayed in FIG. 15, a 'Beamforming' mode of operation of the hearing system is selected (e.g. by the user via the 'EarEOG APP'). The user interface is configured to show which of the multitude of sound sources (S₁, S₂, S₃) that the user is listening to, e.g. selected via eye gaze as proposed in the present disclosure. This is illustrated in the presented screen of the *EarEOG APP* in that a beamformer of the hearing system is directed towards one of the sound sources (here S1). The available sound sources shown by the user interface may in another mode of operation of the hearing system represent wireless reception from one (or more) of a number of audio sources that wirelessly transmits their respective audio signals to the hearing system (cf. e.g. 'Active wireless receiver' mode in FIG. 14 (representing the scenario of FIG. 7).

In the embodiment of FIG. 15, the available wireless links are denoted *IA- WL* (e.g. an inductive link between the hearing devices (*HD_{left}, HD_{right}*)) and *AD-WL(l)* and *AD-WL(r)* (e.g. RF-links between the auxiliary device /AD) and the left and between the auxiliary device and the right hearing device, respectively). The wireless interfaces are implemented in the left and right hearing devices (*HD_{left}, HD_{right}*) by antenna and transceiver circuitry (*(Rx1*/*Tx1)ₗ, (Rx2*/*Tx2)ₗ*) and (*(Rx1*/*Tx1)ᵣ, (Rx2*/*Tx2)ᵣ),* respectively. The different wirless links may be used ensure a stable availability of the relevant data (audio and or informant/control) in the respective devices. The auxiliary device (*AD*) comprising the user interface (*UI*) is e.g. adapted for being held in a hand (*Hand*) of a user (*U*), and hence convenient for displaying information to the user and to be used by the user for controlling the system.

The application program *EarEOG APP* displays currently present sound sources (*S₁, S₂, S₃*) and their estimated localization relative to the user (*U*) as selected by eye gaze. Such system may be combined with other ways of estimating a user's currently preferred audio source, e.g. by correlating captured EEG signals (using the bio sensors of the hearing devices) and the individual, currently present sound source signals (as e.g. provided by a source separation algorithm of the hearing device(s) or the auxiliary device). Such scheme for (automatic) correlation of brainwave signals and current sound source signals is e.g. dealt with in US2014098981A1, wherein coherence between the measured brainwaves and an audio signal picked up by and processed by a forward path of the hearing device(s) (or the auxiliary device) is determined. The determination of the sound source of current interest of the user based on audio signals and brainwave signals may e.g. be performed in the respective hearing devices and the results transmitted to the auxiliary device for comparison (evaluation) and display. Alternatively, the calculations may be performed in the auxiliary device to save power in the hearing devices.

Alternatively or additionally, the selection by eye gaze may be combined with (or overrided by) a manual selection of a sound source (e.g. *S₂)* currently having the attention of the user (thereby overriding the sound source *S₁* determined by eye gaze). A manual selection (and/or deselection) may e.g. be performed via the user interface (*UI*), e.g. by touching the source of interest in question (e.g. *S₂)* on the display. Alternatively or additionally, a manual selection of a source of interest may be used to *add* a further sound source of interest, so that the user at the same time receives audio signals from two or more of the sound sources, e.g. *S₁* and *S₃* in the shown scenario.

In an embodiment, the user interface is configured to allow a user to control the volume (sound level) of the received sound source, and if more than one sound source is selected to control a relative strength of the volumes of the selected sound sources.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

### REFERENCES

- [Bailey & Durrant-Whyte; 2006] Tim Bailey, Hugh Durrant-Whyte, Simultaneous Localization and Mapping (SLAM). IEEE Robotics & Automation Magazine, Part I: June 2006, pp. 99-108, Part II: September 2006, pp. 108-117.
- [Berg & Scherg; 1991] Berg, P., Scherg, M. Dipole models of eye movements and blinks. Electroencephalography and clinical Neurophysiology, 79, 36-44 (1991).
- [Carpenter; 1988] Carpenter, R. H. Movements of the eyes (2nd rev). Pion Limited (1988).
- EP2997893A1 (Oticon) 23.03.2016
- EP3013070A2 (Oticon) 27.04.2016.
- [Harland et al.; 2002] Harland C, Clark T.D., Prance R.J., J. Meas. Sci. and Technol. 13, 163-169 (2002).
- [Hart et al., 2009] Jamie Hart, Dumitru Onceanu, Changuk Sohn, Doug Wightman, and Roel Vertegaal, The Attentive Hearing Aid: Eye Selection of Auditory Sources for Hearing Impaired Users, in T. Gross et al. (eds.) INTERACT 2009, Part I, LNCS5726, pp. 19-35 (2009).
- [Kidd et al.; 2013] Kidd, G., Favrot, S., Desloge, J. G., Streeter, T. M., and Mason, C. R., Design and preliminary testing of a visually guided hearing aid. The Journal of the Acoustical Society of America, 133(3), EL202. doi:10.1121/1.4791710 (2013).
- [Komogortsev & Khan; 2009] Oleg V. Komogortsev, Javed I. Khan, Eye movement prediction by oculomotor plant Kalman filter with brainstem control, J Control Theory Appl., Vol. 7 (1), pp- 14-22 (2009).
- [Kuipers; 2000] Jack B. Kuipers, Quaternions And Rotation Sequences, Geometry, Integrability and Quantization, September 1-10, 1999, Varna, Bulgaria, lvarlo M. Mladenov and Gregory L. Naber, Editors, Coral Press, Sofia 2000, pp 127-143.
- [Ljung; 1999] Ljung L. System Identification. Theory for the User (2nd ed.). New Jersey: Prentice Hall (1999).
- [Manabe & Fukamoto; 2010] H. Manabe, M. Fukamoto, Using earphones to Perform Gaze Detection for Wearable Interfaces, NTT Docomo Technical Journal, Vol. 12, No. 3, pp. 12-17 (2010).
- [Prance et al.; 1997] Prance R J, Clark T D, Prance H and Nock M. Electrometer arrays: sensing of spatio-temporal ELF fields Proc. Marelec (London, 1997) p 3.4.
- [Schaub; 2008] Arthur Schaub, Digital hearing Aids, Thieme Medical. Pub. (2008).
- [Severo & Gama; 2010] M Severo, J Gama, Change detection with Kalman filter and CUSOM, Chapter Ubiquitous Knowledge Discovery, Volume 6202 of the series Lecture Notes in Computer Science, pp 148-162, Springer Berlin Heidelberg (2010).
- Statistical Sensor Fusion - Lab 2, Orientation Estimation using Smartphone Sensors, (https://www.control.isy.liu.se/student/tsrt14/file/orientation.pdf) Automatic Control, Linköping, May 2014, 19 pages
- US20140198936A1 (Starkey Laboratories) 17.07.2014
- US2014098981A1 (Oticon) 10.04.2014.
- US2014369537A1 (OticonA/S) 18.12.2014.

## Claims

1. A hearing system comprising
• a hearing device (HD) comprising
∘ a beanformer filtering unit (BF),
∘ a sensor part adapted for being located at or in an ear and/or for fully or partially being implanted in the head of a user (U), the sensor part comprising,
▪ an electrical potential sensor (EPS) for sensing an electrical potential P, and
∘ electronic circuitry coupled to the electrical potential sensor part to provide an amplified output, and
• an auxiliary device, wherein the hearing device (HD) and the auxiliary device are configured to allow an exchange of data between them, including said amplified output, or signals based thereon, wherein said amplified output, or signals based thereon is/are representative of EEG and/or EOG signals, and **characterized in that** the hearing system further comprises
• a location sensor unit (LSU) for providing location data (LOCD) representative of a current location of the user (U), and
• a calculation unit (CALC) configured to combine said location data (LOCD) with said EEG and/or EOG signal to provide combined location data, wherein said calculation unit is configured
∘ to determine locations of hotspots representing preferred eye gaze directions of the user based on said combined location data, and
∘ to identify a spatial map of currently interesting hotspots;
∘ to use said spatial map of currently interesting hotspots to simplify processing by calculating fixed beamformers of the beamformer filtering unit (BF) for at least some of the identified hotspots to be applied when a given hotspot is estimated to be of the user's current interest, according to the combined location data.

2. A hearing system according to claim 1 wherein the auxiliary device comprises a further hearing device (HD) comprising
∘ a sensor part adapted for being located at or in an ear and/or for fully or partially being implanted in the head of a user, the sensor part comprising,
▪ an electrical potential sensor for sensing an electrical potential P, and
∘ electronic circuitry coupled to the electrical potential sensor part to provide an amplified output,
the hearing system thereby comprising left and right hearing devices (HD₁, HD₂; HD_{left}, HD_{right}) adapted for being located at or in left and right ears and/or for fully or partially for being implanted in the head at left and right ears of a user, and wherein the hearing system is configured to allow an exchange of data between the left and right hearing devices, either directly or via a further auxiliary device, e.g. a remote control or a smartphone or the like.

3. A hearing system according to claim 2 comprising a further auxiliary device (AD) and wherein said further auxiliary device (AD) comprises a user interface (UI) and optionally a further processing capability.

4. A hearing system according to any one of claims 1-3 wherein the electrical potential sensor comprises a sensing electrode configured to be capacitively coupled to the surface of the user's head, when the hearing device is operatively mounted on the user.

5. A hearing system according to any one of claims 1-4 wherein said electronic circuitry provides a reference potential P₀.

6. A hearing system according to claim 5 wherein said electronic circuitry comprises a low noise amplifier arranged to amplify said electrical potential P relative to said reference potential P₀ to provide said amplified output in the form of an amplified voltage A(P-P₀), where A is an amplification factor.

7. A hearing system according to any one of claims 1-6 wherein said hearing device (HD) comprises a hearing aid, a headset, an earphone, an ear protection device or a combination thereof.

8. A hearing system according to any one of claims 2-8 configured to sense over time, a) left and right EPS sense potentials P_{left} and P_{right}, respectively, and b) to compare the sensed potentials to a reference potential P₀, and c) to provide respective amplified voltages V_{left} = A(P_{left} - P₀) and V_{right} = A(P_{right} - P₀), where A is an amplification factor.

9. A hearing system according to claim 8 wherein the left and right amplified voltages V_{left} and V_{right} are representative of respective left and right EEG signals, termed EarEEG_{left} and EarEEG_{right}, respectively, and wherein - in an eye gaze detection mode - the measured potentials V_{left} and V_{right} are representative of eye movement, and wherein the hearing system is configured to transmit one of the left and right amplified voltages V_{left} and V_{right} to the respective other hearing device (HD₁, HD₂; HD_{left}, HD_{right}), or to another device (AD), or to exchange said amplified voltages between the left and right hearing devices (HD₁, HD₂; HD_{left}, HD_{right}), or to transmit said amplified voltages to another device (AD).

10. A hearing system according to claim 9 configured to provide that an EOG signal representative of an eye gaze direction is determined based on the left and right amplified voltages V_{left} and V_{right}.

11. A hearing system according to any one of claims 1-10 comprising a processing unit (PU) configured to provide an EOG control signal for controlling a function of said hearing device (HD) based on said EOG signal(s).

12. A hearing system according to claim 11 configured to allow the reception of audio signals from a multitude of audio sources (S1, ..., S5), and comprising a control unit (CONT) for selecting one of the audio signals in dependence of said EOG control signal(s).

13. A hearing system according to claims 11 or 12 wherein said processing unit (PU) is configured to control the beamformer filtering unit (BF), or a binaural beamformer unit, in dependence of said EOG control signal(s).

14. A hearing system according to any one of claims 1-13 wherein said location sensor unit (LSU) comprises a head tracker based on linear acceleration and/or angular acceleration data for providing said location data (LOCD) representative of a current location (rᵤ) of the user (U).

15. A hearing system according to any one of claims 1-14 comprising a Kalman filter (FIL; KF) for filtering said location data (LOCD), and/or said EEG and/or EOG signal(s), and/or said combined location data, and providing eye gaze angles (θ) or eye gaze directions (EyeGD) in a fixed coordinate system (x, y, z).

16. A hearing system according to any one of claims 1-15 comprising a linear adaptive filter (KF) and a nonlinear change detector (CUSUM) configured to be used for filtering of said amplified output, or signals based thereon.

17. A hearing system according to any one of claims 1-16 wherein said calculation unit (CALC; CALC-FIL) is configured to determine the locations of hotspots representing preferred eye gaze directions (EyeGD) of the user (U) based on filtered location data.

18. A hearing system according to any one of claims 1-17 wherein said calculation unit (CALC; CALC-FIL) is configured to determine said spatial map of currently interesting hotspots for the user by Kalman filtering and Simultaneous Location And Mapping (SLAM) algorithm.

## Patentansprüche

1. Hörsystem, umfassend
• ein Hörgerät (hearing device - HD), umfassend
∘ eine Strahlformerfiltereinheit (beamformer filtering unit - BF),
∘ einen Sensorteil, der dazu angepasst ist, an oder in einem Ohr angeordnet zu sein und/oder vollständig oder teilweise in dem Kopf eines Benutzers (user - U) implantiert zu sein, wobei der Sensorteil Folgendes umfasst:
• einen elektrischen Potenzialsensor (electrical potential sensor - EPS) zum Erfassen eines elektrischen Potenzials P, und
∘ elektronische Schaltung, die mit dem elektronischen Potenzialsensorteil gekoppelt ist, um einen verstärkten Ausgang bereitzustellen, und
• eine Hilfsvorrichtung, wobei das Hörgerät (HD) und die Hilfsvorrichtung dazu konfiguriert sind, einen Austausch von Daten zwischen diesen zu ermöglichen, darunter der verstärkte Ausgang, oder von Signalen basierend darauf, wobei der verstärkte Ausgang, oder Signale basierend darauf, EEG- und/oder EOG-Signale darstellt/darstellen, und **dadurch gekennzeichnet, dass** das Hörsystem ferner Folgendes umfasst:
• eine Standortsensoreinheit (location sensor unit - LSU) zum Bereitstellen von Standortdaten (location data - LOCD), die einen aktuellen Standort des Benutzers (U) darstellen, und
• eine Berechnungseinheit (calculation unit - CALC), die dazu konfiguriert ist, die Standortdaten (LOCD) mit dem EEG- und/oder EOG-Signal zu kombinieren, um kombinierte Standortdaten bereitzustellen, wobei die Berechnungseinheit zu Folgendem konfiguriert ist:
∘ Standorte von Hotspots, die bevorzugte Blickrichtungen eines Auges des Benutzers darstellen, basierend auf den kombinierten Standortdaten zu bestimmen, und
∘ eine räumliche Karte von aktuell interessierenden Hotspots zu identifizieren;
∘ die räumliche Karte von aktuell interessierenden Hotspots zu verwenden, um ein Verarbeiten mittels Berechnen fester Strahlformer der Strahlformerfiltereinheit (BF) für zumindest einige der identifizierten Hotspots, die anzuwenden sind, wenn für einen gegebenen Hotspot geschätzt wird, dass er für den Benutzer von aktuellem Interesse ist, gemäß den kombinierten Standortdaten zu vereinfachen.

2. Hörsystem nach Anspruch 1, wobei die Hilfsvorrichtung ein weiteres Hörgerät (HD) umfasst, das Folgendes umfasst:
∘ einen Sensorteil, der dazu angepasst ist, an oder in einem Ohr angeordnet zu sein und/oder vollständig oder teilweise in dem Kopf eines Benutzers implantiert zu sein, wobei der Sensorteil Folgendes umfasst:
• einen elektrischen Potenzialsensor zum Erfassen eines elektrischen Potenzials P, und
∘ elektronische Schaltung, die mit dem elektronischen Potenzialsensorteil gekoppelt ist, um einen verstärkten Ausgang bereitzustellen,
wodurch das Hörsystem ein linkes und ein rechtes Hörgerät (HD₁, HD₂; HDₗᵢₙₖₛ, HD_{rechts}) umfasst, das dazu angepasst ist, an oder in einem linken oder einem rechten Ohr angeordnet zu sein und/oder vollständig oder teilweise in dem Kopf an dem linken oder dem rechten Ohr eines Benutzers implantiert zu sein, und wobei das Hörsystem dazu konfiguriert ist, einen Austausch von Daten zwischen dem linken und der rechten Hörgerät entweder direkt oder über eine weitere Hilfsvorrichtung, z. B. eine Fernsteuerung oder ein Smartphone oder dergleichen, zu ermöglichen.

3. Hörsystem nach Anspruch 2, umfassend eine weitere Hilfsvorrichtung (auxiliary device - AD) und wobei die weitere Hilfsvorrichtung (AD) eine Benutzerschnittstelle (user interface - UI) und optional eine weitere Verarbeitungsfähigkeit umfasst.

4. Hörsystem nach einem der Ansprüche 1-3, wobei der elektrische Potenzialsensor eine Erfassungselektrode umfasst, die dazu konfiguriert ist, kapazitiv mit der Oberfläche des Kopfes des Benutzers gekoppelt zu sein, wenn das Hörgerät an dem Benutzer wirkbefestigt ist.

5. Hörsystem nach einem der Ansprüche 1-4, wobei die elektronische Schaltung ein Referenzpotenzial Po bereitstellt.

6. Hörsystem nach Anspruch 5, wobei die elektronische Schaltung einen rauscharmen Verstärker umfasst, der dazu angeordnet ist, das elektrische Potenzial P relativ zu dem Referenzpotenzial Po zu verstärken, um den verstärkten Ausgang in der Form einer verstärkten Spannung A(P-Po) bereitzustellen, wobei A ein Verstärkungsfaktor ist.

7. Hörsystem nach einem der Ansprüche 1-6, wobei das Hörgerät (HD) eine Hörhilfe, ein Headset, einen Ohrhörer, eine Ohrschutzvorrichtung oder eine Kombination davon umfasst.

8. Hörsystem nach einem der Ansprüche 2-8, das dazu konfiguriert ist, a) ein linkes bzw. ein rechtes EPS-Erfassungspotenzial Pₗᵢₙₖₛ bzw. P_{rechts} im Zeitverlauf zu erfassen und b) die erfassten Potenziale mit einem Referenzpotenzial Po zu vergleichen und c) entsprechende verstärkte Spannungen Vₗᵢₙₖₛ = A(Pₗᵢₙₖₛ - P₀) und V_{rechts} = A(Prechts - P₀) bereitzustellen, wobei A ein Verstärkungsfaktor ist.

9. Hörsystem nach Anspruch 8, wobei die linke und die rechte verstärkte Spannung Vₗᵢₙₖₛ bzw. V_{rechts} ein entsprechendes linkes bzw. rechtes EEG-Signal, bezeichnet als EarEEGₗᵢₙₖₛ bzw. EarEEGrechts, darstellen, und wobei in einem Erkennungsmodus für den Blick eines Auges die gemessenen Potenziale Vₗᵢₙₖₛ und V_{rechts} eine Augenbewegung darstellen, und wobei das Hörsystem dazu konfiguriert ist, eine von der linken und der rechten verstärkten Spannung Vₗᵢₙₖₛ und V_{rechts} an das entsprechende andere Hörgerät (HD₁, HD₂; HDₗᵢₙₖₛ, HD_{rechts}) oder an eine andere Vorrichtung (AD) zu übertragen, oder die verstärkten Spannungen zwischen dem linken und dem rechten Hörgerät (HD₁, HD₂; HDₗᵢₙₖₛ, HD_{rechts}) auszutauschen oder die verstärkten Spannungen an eine andere Vorrichtung (AD) zu übertragen.

10. Hörsystem nach Anspruch 9, das dazu konfiguriert ist, dafür zu sorgen, dass ein EOG-Signal, das eine Blickrichtung eines Auges darstellt, basierend auf der linken und der rechten verstärkten Spannung Vₗᵢₙₖₛ und V_{rechts} bestimmt wird.

11. Hörsystem nach einem der Ansprüche 1-10, umfassend eine Verarbeitungseinheit (processing unit - PU), die dazu konfiguriert ist, ein EOG-Steuersignal zum Steuern einer Funktion des Hörgeräts (HD) basierend auf dem/den EOG-Signal(en) bereitzustellen.

12. Hörsystem nach Anspruch 11, das dazu konfiguriert ist, den Empfang von Audiosignalen von einer Vielzahl von Audioquellen (S1, ..., S5) zu ermöglichen, und umfassend eine Steuereinheit (control unit - CONT) zum Auswählen eines der Audiosignale in Abhängigkeit von dem/den EOG-Steuersignal(en).

13. Hörsystem nach Anspruch 11 oder 12, wobei die Verarbeitungseinheit (PU) dazu konfiguriert ist, die Strahlformerfiltereinheit (BF) oder einer binaurale Strahlformereinheit in Abhängigkeit von dem/den EOG-Steuersignal(en) zu steuern.

14. Hörsystem nach einem der Ansprüche 1-13, wobei die Standortsensoreinheit (LSU) einen Kopfverfolger basierend auf linearen und/oder winkligen Beschleunigungsdaten zum Bereitstellen der Standortdaten (LOCD), die einen aktuellen Standort (ru) des Benutzers (U) darstellen, umfasst.

15. Hörsystem nach einem der Ansprüche 1-14, umfassend einen Kalman-Filter (FIL; KF) zum Filtern der Standortdaten (LOCD) und/oder des/der EEG- und/oder EOG-Signals/Signale und/oder der kombinierten Standortdaten, und zum Bereitstellen von Blickwinkeln eines Auges (θ) oder Blickrichtungen eines Auges (EyeGD) in einem festen Koordinatensystem (x, y, z).

16. Hörsystem nach einem der Ansprüche 1-15, umfassend einen linearen adaptiven Filter (KF) und einen nichtlinearen Änderungsdetektor (CUSUM), dazu konfiguriert, zum Filtern des verstärkten Ausgangs oder von Signalen basierend darauf verwendet zu werden.

17. Hörsystem nach einem der Ansprüche 1-16, wobei die Berechnungseinheit (CALC; CALC-FIL) dazu konfiguriert ist, die Standorte von Hotspots zu bestimmen, die bevorzugte Blickrichtungen eines Auges (EyeGD) des Benutzers (U) basierend auf gefilterten Standortdaten darstellen.

18. Hörsystem nach einem der Ansprüche 1-17, wobei die Berechnungseinheit (CALC; CALC-FIL) dazu konfiguriert ist, die räumliche Karte von aktuell interessierenden Hotspots für den Benutzer mittels Kalman-Filterung und eines Simultaneous Location And Mapping (Simultane Positionsbestimmung und Kartenerstellung - SLAM)-Algorithmus zu bestimmen.

## Revendications

1. Système auditif comprenant
• un dispositif auditif (HD) comprenant
∘ une unité de filtrage de formateur de faisceaux (BF),
∘ une partie capteur adaptée pour être positionnée au niveau d'une oreille ou dans celle-ci et/ou pour être implantée totalement ou partiellement dans la tête d'un utilisateur (U), la partie capteur comprenant,
• un capteur de potentiel électrique (EPS) pour détecter un potentiel électrique P, et
∘ un ensemble de circuits électroniques couplés à la partie capteur de potentiel électrique pour fournir une sortie amplifiée, et
• un dispositif auxiliaire, ledit dispositif auditif (HD) et ledit dispositif auxiliaire étant configurés pour permettre un échange de données entre eux, comprenant ladite sortie amplifiée, ou des signaux basés sur celle-ci, ladite sortie amplifiée, ou lesdits signaux basés sur celle-ci étant représentatifs de signaux d'électroencéphalographie (EEG) et/ou d'électro-oculographie (EOG), et **caractérisé en ce que** le système auditif comprend en outre
• une unité de capteur de position (LSU) pour fournir des données de position (LOCD) représentatives d'une position actuelle de l'utilisateur (U), et
• une unité de calcul (CALC) configurée pour combiner lesdites données de position (LOCD) avec ledit signal EEG et/ou EOG pour fournir des données de position combinées, ladite unité de calcul étant configurée
∘ pour déterminer des positions de zone sensible représentant les directions préférées du regard de l'utilisateur sur la base desdites données de position combinées, et
∘ pour identifier une carte spatiale des zones sensibles actuellement d'intérêt ;
∘ pour utiliser ladite carte spatiale des zones actuellement d'intérêt afin de simplifier le traitement en calculant des formateurs de faisceaux fixes de l'unité de filtrage de formateur de faisceaux (BF) pour au moins certaines des zones sensibles identifiées à appliquer lorsqu'une zone sensible donnée est estimée être de l'intérêt actuel de l'utilisateur, selon les données de position combinées.

2. Système auditif selon la revendication 1, ledit dispositif auxiliaire comprenant un dispositif auditif (HD) supplémentaire comprenant
∘ une partie capteur adaptée pour être positionnée au niveau d'une oreille ou dans celle-ci et/ou pour être implantée totalement ou partiellement dans la tête d'un utilisateur, la partie capteur comprenant,
• un capteur de potentiel électrique pour détecter un potentiel électrique P, et
∘ un ensemble de circuits électroniques couplés à la partie capteur de potentiel électrique pour fournir une sortie amplifiée,
le système auditif comprenant ainsi des dispositifs auditifs gauche et droit (HD₁, HD₂, HD_{gauche}, HDdroit) adaptés pour être positionnés au niveau des oreilles gauche et droite ou dans celles-ci et/ou pour être totalement ou partiellement implantées dans la tête au niveau des oreilles gauche et droite d'un utilisateur, et ledit système auditif étant configuré pour permettre un échange de données entre les dispositifs auditifs gauche et droit, soit directement, soit par l'intermédiaire d'un dispositif auxiliaire supplémentaire, par exemple une télécommande ou un smartphone ou similaire.

3. Système auditif selon la revendication 2, comprenant un dispositif auxiliaire (AD) supplémentaire et ledit dispositif auxiliaire (AD) supplémentaire comprenant une interface utilisateur (UI) et éventuellement une capacité de traitement supplémentaire.

4. Système auditif selon l'une quelconque des revendications 1 à 3, ledit capteur de potentiel électrique comprenant une électrode de détection configurée pour être couplée capacitivement à la surface de la tête de l'utilisateur, lorsque le dispositif auditif est monté fonctionnellement sur l'utilisateur.

5. Système auditif selon l'une quelconque des revendications 1 à 4, ledit ensemble de circuits électroniques fournissant un potentiel de référence Po.

6. Système auditif selon la revendication 5, ledit ensemble de circuits électroniques comprenant un amplificateur à faible bruit agencé pour amplifier ledit potentiel électrique P par rapport audit potentiel de référence Po pour fournir ladite sortie amplifiée sous la forme d'une tension amplifiée A(P-Po), où A est un facteur d'amplification.

7. Système auditif selon l'une quelconque des revendications 1 à 6, ledit dispositif auditif (HD) comprenant une aide auditive, un casque, un écouteur, un dispositif de protection auditive ou une combinaison de ceux-ci.

8. Système auditif selon l'une quelconque des revendications 2 à 8, configuré pour détecter dans le temps, a) les potentiels de détection EPS gauche et droit P_{gauche} et P_{droit}, respectivement, et b) pour comparer les potentiels détectés à un potentiel de référence P₀, et c) pour fournir des tensions amplifiées respectives V_{gauche} = A(P_{gauche}-P₀) et V_{droite} = A(P_{droit}-P₀), où A est un facteur d'amplification.

9. Système auditif selon la revendication 8, lesdites tensions amplifiées gauche et droite V_{gauche} et V_{droite} étant représentatives des signaux EEG gauche et droit respectifs, appelés respectivement OreilleEEG_{gauche} et OreilleEEGdroite, et - dans un mode de détection du regard - les potentiels mesurés V_{gauche} et V_{droit} étant représentatifs du mouvement des yeux et ledit système auditif étant configuré pour transmettre l'une des tensions amplifiées gauche et droite V_{gauche} et V_{droite} à l'autre dispositif auditif respectif (HD₁, HD₂, HD_{gauche}, HD_{droit}) ou à un autre dispositif (AD), ou pour échanger lesdites tensions amplifiées entre les dispositifs auditifs gauche et droit (HD₁, HD₂, HD_{gauche}, HD_{droit}), ou pour transmettre lesdites tensions amplifiées à un autre dispositif (AD).

10. Système auditif selon la revendication 9 configuré pour assurer qu'un signal EOG représentatif d'une direction de regard est déterminé sur la base des tensions amplifiées gauche et droite V_{gauche} et V_{droite}.

11. Système auditif selon l'une quelconque des revendications 1 à 10, comprenant une unité de traitement (PU) configurée pour fournir un signal de commande EOG pour commander une fonction dudit dispositif auditif (HD) sur la base dudit ou desdits signaux EOG.

12. Système auditif selon la revendication 11 configuré pour permettre la réception de signaux audio provenant d'une multitude de sources audio (S1,..., S5), et comprenant une unité de commande (CONT) pour sélectionner l'un des signaux audio en fonction dudit ou desdits signaux de commande EOG.

13. Système auditif selon les revendications 11 ou 12, ladite unité de traitement (PU) étant configurée pour commander l'unité de filtrage de formateur de faisceau (BF), ou une unité de formateur de faisceau binaural, en fonction dudit ou des signaux de commande EOG.

14. Système auditif selon l'une quelconque des revendications 1 à 13, ladite unité de capteur de position (LSU) comprenant un suiveur de tête basé sur des données d'accélération linéaire et/ou d'accélération angulaire pour fournir lesdites données de position (LOCD) représentatives d'une position actuelle (ru) de l'utilisateur (U).

15. Système auditif selon l'une quelconque des revendications 1 à 14, comprenant un filtre de Kalman (FIL, KF) pour filtrer lesdites données de position (LOCD), et/ou lesdits signaux EEG et/ou EOG, et/ou lesdites données de position combinées et pour fournir des angles de regard (θ) ou des directions de regard (OeilGD) dans un système de coordonnées fixes (x, y, z).

16. Système auditif selon l'une quelconque des revendications 1 à 15, comprenant un filtre adaptatif linéaire (KF) et un détecteur de changement non linéaire (CUSUM) configuré pour être utilisés pour filtrer ladite sortie amplifiée, ou les signaux basés sur celle-ci.

17. Système auditif selon l'une quelconque des revendications 1 à 16, ladite unité de calcul (CALC, CALC-FIL) étant configurée pour déterminer les positions des zones sensibles représentant les directions préférées du regard (OeilGD) de l'utilisateur (U) sur la base de données de position filtrées.

18. Système auditif selon l'une quelconque des revendications 1 à 17, ladite unité de calcul (CALC, CALC-FIL) étant configurée pour déterminer ladite carte spatiale des zones sensibles actuellement d'intérêt pour l'utilisateur par filtrage de Kalman et un algorithme de positionnement et de cartographie simultanées (SLAM).
